Europäisches Patentamt

(19) European Patent Office

Office européen des brevets

(11) EP 0 711 831 A2

(12) EUROPEAN PATENT APPLICATION

(43) Date of publication:
15.05.1996 Bulletin 1996/20

(51) Int. Cl.[6]: C12N 15/12, C07K 14/705, C07K 16/28, G01N 33/68

(21) Application number: 95117786.4

(22) Date of filing: 11.11.1995

(84) Designated Contracting States:
AT BE CH DE DK ES FR GB GR IE IT LI LU NL PT SE

(30) Priority: 14.11.1994 JP 279545/94
24.08.1995 JP 215798/95

(71) Applicant: Takeda Chemical Industries, Ltd.
Osaka-shi, Osaka 541 (JP)

(72) Inventors:
• Hinuma, Shuji
  Tsukuba, Ibaraki 305 (JP)
• Fuji, Ryo
  Tsukuba, Ibaraki 305 (JP)
• Kawamata, Yuji
  Tsukuba, Ibaraki 305 (JP)

(74) Representative: von Kreisler, Alek, Dipl.-Chem. et al
Patentanwälte,
von Kreisler-Selting-Werner,
Bahnhofsvorplatz 1 (Deichmannhaus)
50667 Köln (DE)

(54) Rabbit G protein coupled receptor

(57) Rabbit gastropyrolic part smooth muscle-derived G protein coupled receptor proteins, partial peptides thereof; and DNAs containing said protein or partial peptide-encoding DNA are provided. The receptor protein and the DNA coding for said protein can be used for ① determination of ligands; ② acquisition of antibody and antiserum; ③ construction of expression system for of a recombinant type receptor protein; ④ development of the receptor binding assay system using said expression system and screening of the candidate compounds for pharmaceuticals; ⑤ conducting a drug design based upon a comparison with structurally analogous ligands and receptors; ⑥ preparation of probes and PCR primers for a gene diagnosis; ⑦ preparation of transgenic animals; and ⑧ preparation of model patient animals deficient in the receptor protein DNA. Elucidation of the structure and property of the G protein coupled receptor is particularly related to the development of unique pharmaceuticals which act on such a system.

EP 0 711 831 A2

**Description**

FIELD OF THE INVENTION

The present invention relates to novel proteins of the class of G protein coupled receptors and fragments thereof; novel DNAs encoding such a G protein coupled receptor protein or DNAs encoding fragment thereof; processes for producing said G protein coupled receptor protein (or fragments thereof); use of said receptor protein (or fragments thereof) and said protein (or fragment thereof)-encoding DNA; a method for determination of a ligand against said receptor protein; a method of measuring the physiological actions of said ligand using a G protein coupled receptor protein-expressing cell or the G protein coupled receptor protein; a screening method for a G protein coupled receptor agonist/antagonist using the G protein coupled receptor protein-expressing cell or G protein coupled receptor protein; a kit for said screening; an agonist or antagonist obtained by said screening method or kit; and a pharmaceutical composition containing said agonist or antagonist.

BACKGROUND OF THE INVENTION

A variety of hormones, neurotransmitters and the like control, regulate or adjust the functions of living bodies via specific receptors located in cell membranes. Many of these receptors mediate the transmission of intracellular signals via activation of a guanine nucleotide-binding protein (hereinafter, sometimes referred to as "G protein") which a certain receptor is coupled to and possess the common (homologous) structures, i.e., seven transmembranes (membrane-spanning regions (domains)). Therefore, such a receptor is generically referred to as "G protein coupled receptor" or "seven transmembrane (membrane-spanning) receptor".

These G protein coupled receptor proteins are widely distributed in functional cellular surface of cells and organs in the living bodies and have a very important role as targets for molecules such as hormones, neurotransmitters and physiologically active substances, which molecules control, regulate or adjust the functions of living bodies.

The digestive organs such as the stomach and small intestine carry out the digestion and absorption of ingested food by secreting a variety of digestive fluids under the regulation of various hormones, hormone-like substances, neurotransmitters, physiologically active substances and the like. It is believed that the secretion of these substances is controlled by receptors, which are each specific to a specific substance. Secretion of various factors, specifically gastrointestinal hormones such as secretin, gastrin, cholecystokinin, vasoactive intestinal peptide, motilin, substance P, somatostatin and neurotensin, responds to physical or chemical stimulation from the gastrointestinal lumen or nervous stimulation; however, most of their actual physiological actions are still unclear. As for motilin, there has not yet been reported any discovery concerning the structure of their receptor protein cDNA. It is not known whether any unknown receptor proteins or receptor protein subtypes exist for motilin.

It is very important in investigating and developing new pharmaceuticals to clarify the relation between substances (controlling the complicated functions of stomach and small intestine) and specific receptors thereto. In order to develop new pharmaceuticals, for example, by conducting an effective screening for agonists and antagonists to the receptor proteins for controlling the functions of stomach and small intestine, it is necessary to investigate the function of receptor protein genes and also to express them in a suitable expression system.

By utilizing the fact that a G protein coupled receptor exhibits homology in part of the structure thereof at the amino acid sequence level, looking at DNAs coding for novel receptor proteins relying upon a polymerase chain reaction (hereinafter simply referred to as "PCR") has recently been done.

SUMMARY OF THE INVENTION

An object of the present invention is to provide novel G protein coupled receptor proteins which are derived from rabbit gastropyrolic part smooth muscles; DNAs comprising a DNA coding for said G protein coupled receptor protein; processes for producing said receptor protein; transformants capable of expressing said receptor protein; cell membrane fractions obtained from said transformant; methods for determining a ligand to the receptor protein; screening methods for a compound or a salt thereof capable of inhibiting the binding of the ligand with the receptor protein; kits for said screening method, pharmaceutical compositions comprising the inhibitory compound; antibodies against said receptor protein; immunoassays using said receptor protein or said antibody and use of said receptor protein and encoding DNA.

Another object of the present invention is to provide novel G protein coupled receptor proteins and fragments thereof or salts thereof; DNAs comprising a DNA coding for said G protein coupled receptor protein or a fragment thereof; vectors carrying said DNA; transformants carrying said vector; cell membrane fractions obtained from said transformant; processes for producing said receptor protein or a fragment thereof, or a salt thereof; methods for determining a ligand to said receptor protein; methods for measuring the physiological actions of the ligand using the G protein coupled receptor protein (including a cell membrane fraction containing the receptor protein) or a G protein coupled receptor protein-expressing cell (including the transformant); screening methods for a G protein coupled receptor agonist/antagonist using the G protein coupled receptor protein or a G protein coupled receptor protein-expressing cell (including the transformant); kits for said screening; agonists or antagonists, obtained by said screening method; pharmaceutical compositions containing said agonist or antagonist; antibodies against said receptor protein; immunoassays using said receptor protein or said antibody; and use of said receptor protein and encoding DNA.

In order to achieve the above-mentioned aims, the present inventors have made extensive investigations. As a result, the present inventors have succeeded in synthesizing DNA primers effective in efficiently isolating DNAs (DNA fragments) coding for G protein coupled receptor proteins by PCR techniques. The present inventors have succeeded in amplifying cDNA derived from various tissues or cells, particularly rabbit gastropyrolic part smooth muscles, with said synthetic DNA primer, and have forwarded the analysis. Thus, the present inventors have succeeded in isolating novel G protein coupled receptor protein-encoding cDNAs from rabbit gastropyrolic part smooth muscles using a synthetic DNA primer for more effective isolation thereof, in determining the partial structure thereof, and have considered that the isolated cDNAs are homologous to known G protein coupled receptors at the nucleotide sequence level and at the amino acid sequence level and are each coding for a novel G protein coupled receptor protein, which is expressed and functions in rabbit stomach. Based upon the above knowledge, the present inventors have discovered that these DNAs make it possible to obtain a cDNA having a full length open reading frame (ORF) of the receptor protein, hence, to produce the receptor protein. The inventors have further succeeded in sequencing an entire amino acid sequence and entire nucleotide sequence of said G protein coupled receptor protein.

The present inventors have found that, when said receptor protein expressed by a suitable means is used, a ligand to said receptor protein can be screened in vivo or from natural or nonnatural compounds by a receptor protein binding experiment or by a measurement of intracellular second messenger, etc. as an index. The present inventors have further found that it is possible to screen for an agonist or antagonist to said receptor protein by a receptor protein binding experiment or by a measurement of intracellular second messenger, etc.

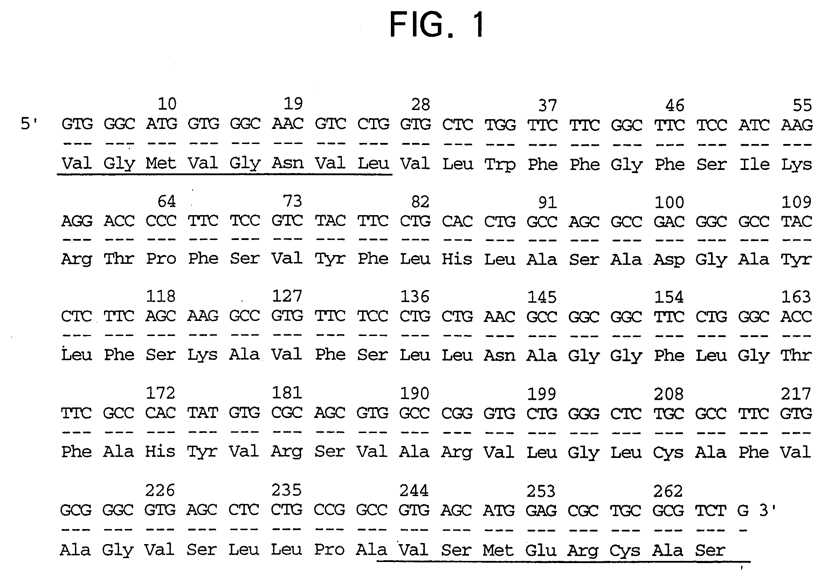

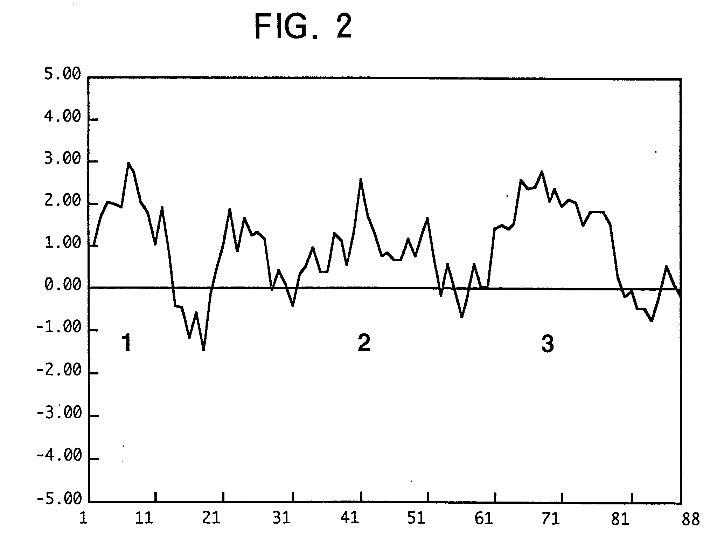

More specifically, the present inventors have amplified novel cDNA fragments derived from rabbit gastropyrolic part smooth muscles as shown in FIG. 1 by PCR and cloned said cDNA fragment in plasmid vectors (pMD4). From the result of analysis of their sequence, the present inventors have clarified that they code for a novel receptor protein. When said sequence was translated into amino acid sequences (FIG. 1), the first, second and third transmembrane domains were confirmed on hyrophobic plots (FIG. 2). The size of the amplified cDNA is about 300 bp which is nearly comparable with the number of bases between the first membrane-spanning domain and the third membrane-spanning domain of the known G protein coupled receptor protein.

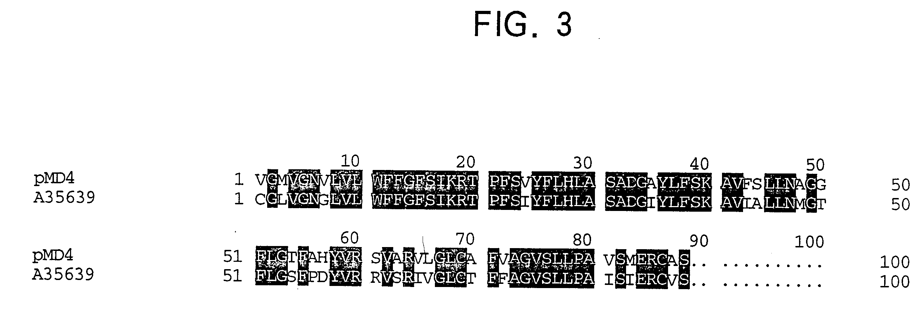

G protein coupled receptor proteins have common properties to some extent at the amino acid sequence level, and form one protein family. Therefore, database retrieval has been conducted based upon the putative amino acid sequence of the subject novel receptor protein (protein encoded by cDNA included in pMD4). As a result, it was found that it had 76% homology relative to the known G protein coupled receptor protein (rat-derived ligand unknown receptor protein (A35639) (FIG. 3). This indicates that the novel receptor protein of the present invention belongs to the G protein coupled receptor protein family. The aforementioned abbreviation in parentheses is a reference number that is assigned when it is registered as data to NBRF-PIR/Swiss-PROT and is, usually, called "Accession Number".

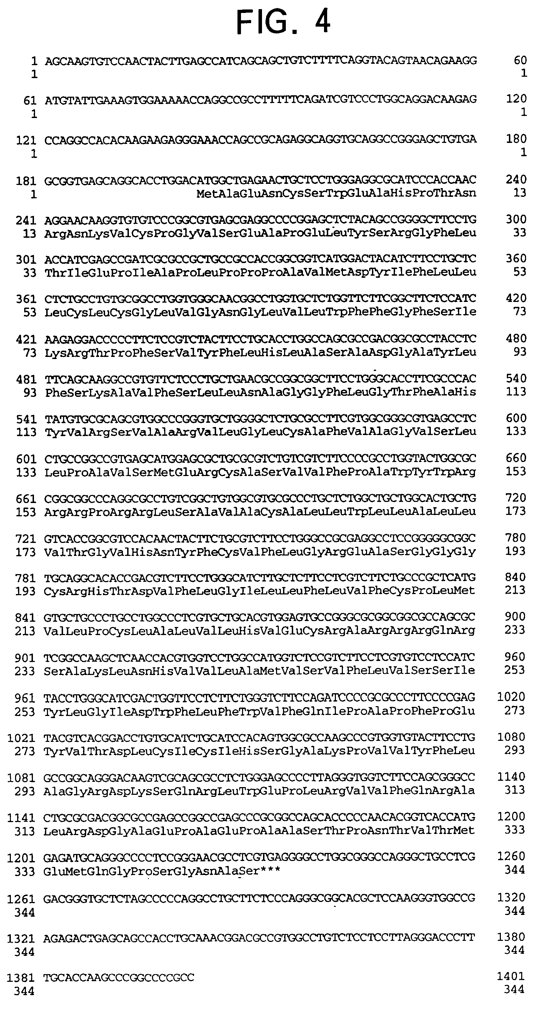

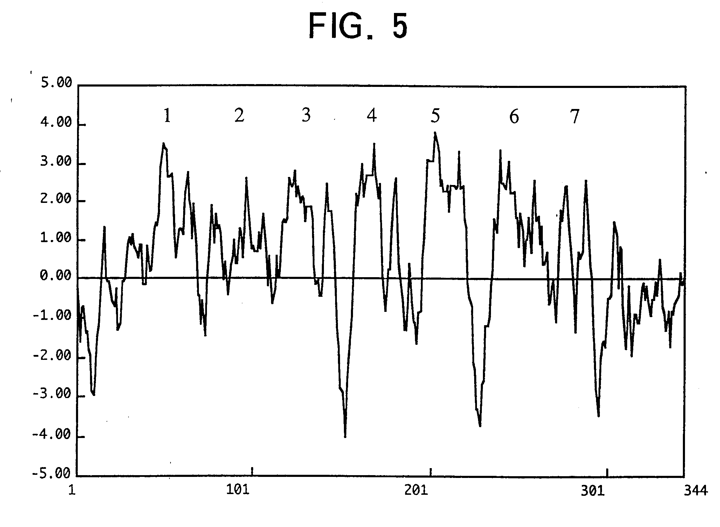

Next, the present inventors have prepared cDNA from the poly(A)⁺RNA fractions extracted from rabbit gastropyrolic part smooth muscles and have inserted said cDNA into lambda gt11 phage to prepare a cDNA library. Further, the present inventors have screened the rabbit gastropyrolic part cDNA library using, as a probe, the novel G protein coupled receptor protein-encoding cDNA fragment (pMD4) obtained by PCR and succeeded in cloning cDNA which has a full-length translation unit (open reading frame; ORF) completely coding for the G protein coupled receptor protein of the present invention. Sequencing of plasmids (pUC-C3) containing the cDNA with a full-length ORF for the receptor protein shows that the nucleotide sequence of a coding region of this receptor protein is represented by SEQ ID NO: 4, and the amino acid sequence deduced therefrom is represented by SEQ ID NO: 2 [FIG. 4]. Based upon the amino acid sequence, hydrophobicity plotting has been conducted. The results are shown in FIG. 5. From the hydrophobicity plotting, it has been clarified that the receptor protein of the present invention possessed seven hydrophobic domains. That is, it has been confirmed that the receptor protein encoded by the cDNA obtained according to the present invention is a seven transmembrane (membrane-spanning) G protein coupled receptor protein.

Accordingly, one aspect of the present invention is

(1) a G protein coupled receptor protein comprising an amino acid sequence selected from the group consisting of an amino acid sequence represented by SEQ ID NO: 1 or SEQ ID NO: 2 and its substantial equivalents thereto, or a salt thereof;
(2) a DNA which comprises a nucleotide sequence coding for a G protein coupled receptor protein according to (1);
(3) the DNA according to (2) comprising a nucleotide sequence represented by SEQ ID NO: 3 or SEQ ID NO: 4;
(4) a vector comprising the DNA according to (2);
(5) a transformant carrying the vector according to (4);
(6) a process for producing a G protein coupled receptor protein or a salt thereof according to (1), which comprises culturing a transformant of (5) to express said G protein coupled receptor protein;

(7) a method for determining a ligand to the G protein coupled receptor protein according to (1), which comprises contacting (i) a G protein coupled receptor protein or a salt thereof according to (1), with (ii) a sample to be tested;

(8) a screening method for a compound capable of inhibiting the binding of a G protein coupled receptor protein according to (1) with a ligand, which comprises making a comparison between:

> (i) at least one case where said ligand is contacted with a G protein coupled receptor protein or a salt thereof according to (1),
> and
> (ii) at least one case where said ligand together with a sample to be tested is contacted with a G protein coupled receptor protein or a salt thereof according to (1);

(9) a kit for the screening of a compound capable of inhibiting the binding of a G protein coupled receptor protein according to (1) with a ligand, which comprises a G protein coupled receptor protein or a salt thereof according to (1);

(10) an antibody against a G protein coupled receptor protein or a salt thereof according to (1); and

(11) a reagent for probing a G protein coupled receptor protein, which comprises a DNA according to (2).

As used herein the term "G protein coupled receptor protein or a salt thereof" refers to the G protein coupled receptor according to the present invention, its salt, a fragment or segment thereof or a salt thereof, a mixture thereof.

Another aspect of the present invention is:

(12) a G protein coupled receptor protein comprising an amino acid sequence selected from the group consisting of an amino acid sequence represented by SEQ ID NO: 1 and its substantial equivalents thereto, or a salt thereof;

(13) a G protein coupled receptor protein comprising an amino acid sequence selected from the group consisting of an amino acid sequence represented by SEQ ID NO: 2 and its substantial equivalents thereto, or a salt thereof;

(14) a fragment of the G protein coupled receptor protein according to (1), (12) or (13), or a salt thereof;

(15) a DNA which comprises a nucleotide sequence coding for the receptor protein of (12);

(16) a DNA which comprises a nucleotide sequence coding for the receptor protein of (13);

(17) a DNA of (15) comprising a nucleotide sequence represented by SEQ ID NO: 3;

(18) a DNA of (16) comprising a nucleotide sequence represented by SEQ ID NO: 4;

(19) a vector comprising a DNA according to (15) or (16);

(20) a transformant (including a transfectant) carrying a vector of (19);

(21) a process for producing a G protein coupled receptor protein or a salt thereof according to (12) or (13), which comprises culturing a transformant of (20) under conditions to express said receptor;

(22) a process for producing a rabbit gastropyrolic part smooth muscle-derived G protein coupled receptor protein or a salt thereof according to (12) or (13), which comprises culturing a transformant of (20) to produce said receptor on the membrane of the transformant;

(23) a method for determining a ligand to a G protein coupled receptor protein according to (12) or (13), which comprises contacting

> (i) at least one component selected from the group consisting of G protein coupled receptor proteins or salts thereof according to (12) or (13) including fragments thereof or salts thereof according to (14), and mixtures thereof,
> with
> (ii) at least one sample to be tested;

(24) a screening method for a compound capable of inhibiting the binding of a G protein coupled receptor protein according to (12) or (13) with a ligand, which comprises comparing:

> (i) at least one case where said ligand is contacted with at least one component selected from the group consisting of G protein coupled receptor proteins or salts thereof according to (12) or (13) above, including fragments or salts thereof according to (14), and mixtures thereof,
> with
> (ii) at least one case where said ligand together with a compound to be tested is contacted with at least one component selected from the group consisting of G protein coupled receptor proteins or salts thereof according to (12) or (13) above, including fragments or salts thereof according to (14), and mixtures thereof;

(25) a kit for the screening of one or more compounds capable of inhibiting the binding of a G protein coupled receptor protein according to (12) or (13), with a ligand, which comprises at least one component selected from the group consisting of G protein coupled receptor proteins or salts thereof according to (12) or (13), including fragments or salts thereof according to (14), and mixtures thereof; and

(26) an antibody against at least one component selected from the group consisting of G protein coupled receptor proteins or salts thereof according to (12) or (13), including fragments or salts thereof according to (14), and mixtures thereof.

Yet another aspect of the present invention is

(27) a G protein coupled receptor protein according to (12) which comprises an amino acid sequence selected from the group consisting of an amino acid sequence represented by SEQ ID NO: 1, amino acid sequences wherein one or more amino acid residues (preferably from 2 to 30 amino acid residues, more preferably from 2 to 10 amino acid residues) are deleted from the amino acid sequence of SEQ ID NO: 1, amino acid sequences wherein one or more amino acid residues (preferably from 2 to 30 amino acid residues, more preferably from 2 to 10 amino acid residues) are added to the amino acid sequence of SEQ ID NO: 1, and amino acid sequences wherein one or more amino acid residues (preferably from 2 to 30 amino acid residues, more preferably from 2 to 10 amino acid residues) in the amino acid sequence of SEQ ID NO: 1 are substituted with one or more other amino acid residues;

(28) a G protein coupled receptor protein according to (13) which comprises an amino acid sequence selected from the group consisting of an amino acid sequence represented by SEQ ID NO: 2, amino acid sequences wherein one or more amino acid residues (preferably from 2 to 30 amino acid residues, more preferably from 2 to 10 amino acid residues) are deleted from the amino acid sequence of SEQ ID NO: 2, amino acid sequences wherein one or more amino acid residues (preferably from 2 to 30 amino acid residues, more preferably from 2 to 10 amino acid residues) are added to the amino acid sequence of SEQ ID NO: 2, and amino acid sequences wherein one or more amino acid residues (preferably from 2 to 30 amino acid residues, more preferably from 2 to 10 amino acid residues) in the amino acid sequence of SEQ ID NO: 2 are substituted with one or more other amino acid residues;

(29) a method for determining a ligand according to (23) wherein said ligand is selected from the group consisting of angiotensin, bombesin, canavinoid, cholecystokinin, glutamine, serotonin, melatonin, neuropeptide Y, opioid, purine, vasopressin, oxytocin, VIP (vasoactive intestinal and related peptide), somatostatin, dopamine, motilin, amylin, bradykinin, CGRP (calcitonin gene related peptide), adrenomedullin, leukotriene, pancreastatin, prostaglandin, thromboxane, adenosine, adrenaline, $\alpha$- and $\beta$-chemokine (IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptide and galanin;

(30) a method for the screening of a compound or a salt thereof capable of inhibiting the binding of a ligand with a G protein coupled receptor protein according to (12) or (13), which comprises measuring amounts of a labeled ligand bound to said G protein coupled receptor protein in at least two cases:

 (i) where the labeled ligand is contacted with at least one component selected from the group consisting of G protein coupled receptor proteins or salts thereof according to (12) or (13), fragments thereof or salts thereof according to (14), and mixtures thereof, and
 (ii) where the labeled ligand together with a compound to be tested is contacted with at least one component elected from the group consisting of G protein coupled receptor proteins or salts thereof according to (12) or (13), fragments thereof or salts thereof according to (14), and mixtures thereof,

and comparing the measured amounts of the labeled ligand;

(31) a method for the screening of a compound or a salt thereof capable of inhibiting the binding of a ligand with a G protein coupled receptor protein according to (12) or (13), which comprises measuring amounts of a labeled ligand bound to a cell comprising said G protein coupled receptor protein in at least two cases:

 (i) where the labeled ligand is contacted with the said cell, and
 (ii) where the labeled ligand together with a compound to be tested is contacted with the said cell,

and comparing the amounts of the labeled ligand measured;

(32) a method for the screening of a compound or a salt thereof capable of inhibiting the binding of a ligand with a G protein coupled receptor protein according to (12) or (13), which comprises measuring amounts of a labeled ligand bound to a membrane fraction of a cell comprising said G protein coupled receptor protein in at least two cases:

 (i) where the labeled ligand is contacted with said membrane fraction, and
 (ii) where the labeled ligand together with a compound to be tested is contacted with the membrane fraction,

and comparing the amounts of the labeled ligand measured;

(33) a method for the screening of a compound or a salt thereof capable of inhibiting the binding of a ligand with a G protein coupled receptor protein according to (12) or (13), which comprises measuring amounts of a labeled ligand bound to said G protein coupled receptor protein in at least two cases:

(i) where the labeled ligand is contacted with a G protein coupled receptor protein according to (12) or (13) which is expressed on the membrane of a transformant according to (20) during incubation of the transformant, and
(ii) where the labeled ligand together with a compound to be tested is contacted with the G protein coupled receptor protein according to (12) or (13) which is expressed on the membrane of a transformant according to (20) during incubation of the transformant,

and comparing the amounts of the labeled ligand measured;
(34) a method for the screening of a compound or a salt thereof capable of inhibiting the binding of a ligand with a G protein coupled receptor protein according to (12) or (13), which comprises measuring G protein coupled receptor protein-mediated cell-stimulating activities in at least two cases:

(i) where a compound capable of activating the G protein coupled receptor protein according to (12) or (13) is contacted with a cell comprising said G protein coupled receptor protein, and
(ii) where the compound capable of activating the G protein together with a compound to be tested is contacted with the cell comprising said G protein coupled receptor protein,

and comparing the cell-stimulating activities measured;
(35) a method for the screening of a compound or a salt thereof capable of inhibiting the binding of a ligand with a G protein coupled receptor protein according to (12) or (13), which comprises measuring G protein coupled receptor protein-mediated cell-stimulating activities in at least two cases:

(i) where a compound capable of activating the G protein coupled receptor protein according to (12) or (13) is contacted with a G protein coupled receptor protein according to (12) or (13) which is expressed on the membrane of a transformant according to (20) during incubation of the transformant, and
(ii) where the compound capable of activating the G protein together with a compound to be tested is contacted with the G protein coupled receptor protein according to (12) or (13) which is expressed on the membrane of a transformant according to (20) during incubation of the transformant,

and comparing the cell-stimulating activities measured;
(36) a method according to (34) or (35) wherein said compound capable of activating the G protein coupled receptor protein according to (12) or (13) is selected from the group consisting of angiotensin, bombesin, canavinoid, cholecystokinin, glutamine, serotonin, melatonin, neuropeptide Y, opioid, purine, vasopressin, oxytocin, VIP (vasoactive intestinal and related peptide), somatostatin, dopamine, motilin, amylin, bradykinin, CGRP (calcitonin gene related peptide), adrenomedullin, leukotriene, pancreastatin, prostaglandin, thromboxane, adenosine, adrenaline, $\alpha$- and $\beta$-chemokine (IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptide and galanin;
(37) a compound which is obtained according to any of the above methods (24) and (30) to (36) or a salt thereof;
(38) a pharmaceutical composition comprising an effective amount of a compound according to (37) or a salt thereof;
(39) a screening kit according to (25), which comprises a cell comprising a G protein coupled receptor protein according to (12) or (13);
(40) a screening kit according to (25), comprising a membrane fraction derived from a cell comprising a G protein coupled receptor protein according to (12) or (13);
(41) a compound which is obtained by means of a screening kit according to any of (25), (39) or (40) or a salt thereof;
(42) a pharmaceutical composition comprising an effective amount of a compound according to (41) or a salt thereof; and
(43) a method for measuring at least one component selected from the group consisting of G protein coupled receptor proteins or salts thereof according to (12) or (13), fragments or salts thereof according to (14), and mixtures thereof, which comprises contacting an antibody according to (26) with the component selected from the group consisting of G protein coupled receptor proteins or salts thereof according to (12) to (13), fragments or salts thereof according to (14), and mixtures thereof.

As used herein the term "sample" refers to any material derived from a subject preferably selected from the group consisting of animal tissues and cells including human tissues and human cells; peptides; proteins; nonpeptidic compounds; synthetic compounds; fermented products; etc. Examples of such human tissues include adrenal, umbilical cord, brain, tongue, liver, lymph gland, lung, thymus, placenta, peritoneum, retina, spleen, heart, smooth muscle, intestine, vessel, bone, kidney, skin, fetus, mammary gland, ovary, testis, pituitary gland, pancreas, submandibular gland, spine, prostate gland, stomach, thyroid gland, trachea (windpipe), skeletal muscle, uterus, adipose tissue, urinary bladder, cornea, olfactory bulb, bone marrow, amnion, etc. Examples of such human cells include nerve cells, epithelial cells, endothelial cells, leukocytes, lymphocytes, gliacytes, fibroblasts, keratinized cells, osteoblasts, osteoclasts, astrocytes, melanocytes, various carcinomas, various sarcomas, various cells derived from the above-mentioned human tissues.

As used herein the term "substantial equivalent(s)" means that an activity characteristic of the protein, e.g., nature of the ligand binding activity, and physical characteristics are substantially the same. For example, the activity would be at least about 75% that of the protein, preferably at least about 85%, more preferably at least about 90%, still more preferably at least about 95%. For example, substitutions, deletions or insertions of amino acids often do not produce radical changes in the physical and chemical characteristics of a polypeptide, in which case polypeptides containing the substitution, deletion, or insertion would be considered to be substantially equivalent to polypeptides lacking the substitution, deletion, or insertion. Substantially equivalent substitutes for an amino acid within the sequence may be selected from other members of the class to which the amino acid belongs, which do not effect the tertiary structure of the protein. The non-polar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine, The positively charged (basic) amino acids include arginine, lysine and histidine. The negatively charged (acidic) amino acids include aspartic acid and glutamic acid.

## BRIEF DESCRIPTION OF THE DRAWINGS

FIG. 1 is the nucleotide sequence (SEQ ID NO: 3) of the rabbit gastropyrolic part smooth muscle-derived G protein coupled receptor protein cDNA fragment included in the novel receptor protein cDNA clone, pMD4, obtained from rabbit gastropyrolic part smooth muscles by PCR amplification, and the amino acid sequence encoded thereby (SEQ ID NO: 1), wherein the underlined parts correspond to the synthetic primers used for the PCR amplification.

FIG. 2 is the hydrophobicity plotting profile of the protein encoded by the rabbit gastropyrolic part smooth muscle-derived G protein coupled receptor protein cDNA fragment included in pMD4, prepared based upon the amino acid sequence shown in FIG. 1, wherein numerals 1 to 3 suggest the presence of hydrophobic domains.

FIG. 3 is the partial amino acid sequence (pMD4) (SEQ ID NO: 1) of the protein encoded by the rabbit gastropyrolic part smooth muscle-derived G protein coupled receptor protein cDNA fragment included in pMD4 as shown in FIG. 1, relative to the known G protein coupled receptor protein, rat ligand unknown receptor protein (A35639), wherein reverse amino acid residues are in agreement, the 1st to 88th amino acid residues of the pMD4 sequence correspond to the 1st to 88th amino acid residues in FIG. 1.

FIG. 4 is the nucleotide sequence of the rabbit gastropyrolic part smooth muscle-derived receptor protein cDNA insert in pUC-C3 (SEQ ID NO: 4), cloned by using as a probe the cDNA insert in pMD4, and the amino acid sequence encoded thereby (SEQ ID NO: 2).

FIG. 5 is the hydrophobicity plotting profile, prepared based upon the amino acid sequence shown in FIG. 4, wherein the axis of ordinate represents an index of hydrophobicity, the axis of abscissa represents the number of amino acids and numerals 1 to 7 represent the presence of hydrophobic domains.

## DETAILED DESCRIPTION OF THE PREFERRED EMBODIMENT

According to the present invention, G protein coupled receptor proteins and fragments thereof or salts thereof; DNAs comprising a DNA coding for said G protein coupled receptor protein or a fragment thereof; vectors carrying said DNA; transformants wherein said vector harbors; cell membrane fractions obtained from said transformant; processes for producing said receptor protein or a fragment thereof, or a salt thereof; methods for measuring the physiological actions of G protein coupled using the G protein coupled receptor protein (including a cell membrane fraction containing the receptor protein) or a G protein coupled receptor protein-expressing cell (including the transformant); screening methods for a G protein coupled receptor agonist/antagonist using the G protein coupled receptor protein or a G protein coupled receptor protein-expressing cell (including the transformant); kits for said screening; agonists or antagonists, obtained by said screening method; pharmaceutical compositions containing said agonist or antagonist; antibodies against said receptor protein; immunoassays using said receptor protein or said antibody; use of said receptor protein and encoding DNA; etc. may be successfully provided. For example, template DNAs coding for part or all of the polypeptide sequence of G protein coupled receptor protein, can be successfully obtained and various DNA sequences encoding part or all of the polypeptide sequence of G protein coupled receptor protein can be isolated and characterized. Further, G protein coupled receptor proteins, fragments derived from the G protein coupled receptor protein, modified derivatives or analogues thereof, and salts thereof are recognized, predicted, deduced, produced, expressed, isolated and characterized. More specifically, DNA sequences comprising each a nucleotide sequence indicated by a SEQ ID NO selected from the group consisting of SEQ ID NO: 3 and SEQ ID NO: 4 have been isolated and characterized. G protein coupled receptor proteins comprising each part or all of an amino acid sequence selected from the group consisting of an amino acid sequence represented by SEQ ID NO: 1 or SEQ ID NO: 2 and substantial equivalents thereto, or a salt thereof.

These G protein coupled receptor proteins are those derived from any cells and tissues (e.g. stomach, pituitary gland, pancreas, brain, kidney, liver, gonad, thyroid gland, cholecyst, bone marrow, adrenal, skin, muscle, lung, digestive duct, blood vessel, heart, etc.) of warm-blooded animals (e.g. guinea pig, rat, mouse, rabbit, swine, sheep, cattle, horse, monkey, human being, cat, dog, etc.), and any G protein coupled receptor proteins as long as they comprise an amino acid sequence selected from the group consisting of an amino acid sequence represented by SEQ ID NO: 1 and an amino acid sequence represented by SEQ ID NO: 2, and substantial equivalents to the amino acid sequence represented by SEQ ID NO: 1 or SEQ ID NO: 2. These G protein coupled receptor proteins may include proteins having an amino acid sequence selected from the group consisting of an amino acid sequence represented by SEQ ID NO: 1 and an amino acid sequence represented by SEQ ID NO: 2, proteins wherein the amino acid sequence thereof is about 90% to 99.9% homologous to the amino acid sequence represented by SEQ ID NO: 1 or the amino acid sequence represented by SEQ ID NO: 2 and the activity thereof is substantially equivalent to the protein having an amino acid sequence represented by SEQ ID NO: 1 or an amino acid sequence represented by SEQ ID NO: 2 and the like. The substantially equivalent activity includes ligand binding activity, signal information transmitting, etc. The term "substantial equivalent" or "substantially equivalent" means that the nature of the ligand binding activity and the like is equivalent, for example, at least about 75% of the protein of SEQ ID NO: 1 or 2, more preferably, at least about 85%, still more preferably, at least about 90%, even more preferably, at least about 95%. Therefore, it is possible that some differences such as ligand binding affinity grades and ligand binding activity grades and quantitative factors such as molecular weights of receptor proteins may exist.

In another embodiment of the present invention, G protein coupled receptor proteins include rabbit gastropyrolic part smooth muscle-derived G protein coupled receptor proteins comprising the amino acid sequence represented by SEQ ID NO: 1, rabbit gastropyrolic part smooth muscle-derived G protein coupled receptor proteins comprising the amino acid sequence represented by SEQ ID NO: 2, etc. Examples of the G protein coupled receptor protein are proteins having the amino acid sequence represented by SEQ ID NO: 1 or SEQ ID NO: 2, proteins wherein one or more amino acid residues (preferably from 2 to 30 amino acid residues, more preferably from 2 to 10 amino acid residues) are deleted from the amino acid sequence of SEQ ID NO: 1 or SEQ ID NO: 2, proteins wherein one or more amino acid residues (preferably from 2 to 30 amino acid residues, more preferably from 2 to 10 amino acid residues) are added to the amino acid sequence of SEQ ID NO: 1 or SEQ ID NO: 2, proteins wherein one or more amino acid residues (preferably from 2 to 30 amino acid residues, more preferably from 2 to 10 amino acid residues) in the amino acid sequence of SEQ ID NO: 1 or SEQ ID NO: 2, are substituted with one or more amino acid residues, etc.

A portion of the amino acid sequence may be modified (e.g. addition, deletion, substitution with other amino acids, etc.) in the G protein coupled receptor proteins of the present invention.

Furthermore, the G protein coupled receptor proteins of the present invention includes those wherein N-terminal Met is protected with a protecting group (e.g., $C_{1-6}$ acyl group such as formyl, acetyl, etc.), those wherein the N-terminal side of Glu is cleaved in vivo to make said Glu pyroglutaminated, those wherein the intramolecular side chain of amino acids is protected with a suitable protecting group (e.g., $C_{1-6}$ acyl group such as formyl, acetyl, etc.), conjugated proteins such as so-called "glycoproteins" wherein saccharide chains are bonded, etc.

The salt of said G protein coupled receptor protein of the present invention preferably includes physiologically acceptable acid addition salts. Examples of such salts are salts thereof with inorganic acids (e.g. hydrochloric acid, phosphoric acid, hydrobromic acid, sulfuric acid, etc.), salts thereof with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), etc.

The G protein coupled receptor protein or its salt of the present invention may be manufactured from the tissues or cells of warm-blooded animals by purifying techniques which are known per se by those skilled in the art or methods similar thereto or may be manufactured by culturing the transformant (or transfectant) (as described herein below) containing G protein coupled receptor protein encoding DNA. The protein or its salt of the present invention may be manufactured by the peptide synthesis as described herein below.

The G protein coupled receptor protein fragment (sometimes also referred as a partial peptide of said G protein coupled receptor protein) may include, for example, a fragment containing an extracellular portion of the receptor, i.e. the site which is exposed outside the cell membranes. Examples of the partial peptide are fragments containing a region which is an extracellular area (hydrophilic region or site) as analyzed in a hydrophobic plotting analysis on the G protein coupled receptor protein, such as shown in FIG. 2 and FIG. 5. A fragment which partly contains a hydrophobic region or site may be used as well. Further, a peptide which separately contains each domain may be used too although a partial peptide (or peptide fragment) which contains multiple domains at the same time will be used as well. This fragment is preferably at least 3 to 50 amino acid residues in length, more preferably at least 3 to 30 amino acid residues, even more preferably at least 3 to 20 amino acid residues. The fragment contains at least 5 amino acids unique to SEQ ID NO: 1 or 2, compared to the corresponding portion of the known G protein coupled receptor protein A35639 (FIG. 3).

The salt of said G protein coupled receptor partial peptide includes preferably physiologically acceptable acid addition salts. Examples of such salts are salts thereof with inorganic acids (e.g. hydrochloric acid, phosphoric acid, hydrobromic acid, sulfuric acid, etc.), salts thereof with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), etc.

The partial peptide of the G protein coupled receptor protein may be manufactured by synthesis methods for peptides known per se by those skilled in the art or methods similar thereto or by cleaving (digesting) G protein coupled receptor proteins by a suitable peptidase. Use of recombinant techniques based upon the present disclosure can also be used. Methods of synthesizing peptide may be any of a solid phase synthesis and a liquid phase synthesis. Thus, a partial peptide (peptide fragment) or amino acids which can construct the protein of the present invention are condensed with the residual part thereof and, when the product has a protective group, said protective group is detached whereupon a desired peptide can be manufactured. Examples of the known methods for condensation and for detachment of protective groups include the following ① to ⑤, which are incorporated herein by reference:

① M. Bodanszky and M. A. Ondetti: Peptide Synthesis, Interscience Publishers, New York (1966).
② Schroeder and Luebke: The Peptide, Academic Press, New York, (1965).
③ Nobuo Izumiya et al.: Fundamentals and Experiments of the Peptide Synthesis, Maruzen KK, Japan (1975).
④ Haruaki Yajima and Shumpei Sakakibara: "Seikagaku Jikken Koza 1" (Experiments of Biochemistry, Part 1), "Tanpakusitu No Kagaku IV" (Chemistry of Protein, IV), p.205 (1977), Japan.
⑤ Haruaki Yajima (ed): Development of Pharmaceuticals (Second Series), Vol. 14, Peptide Synthesis, Hirokawa Shoten, Japan.

After synthesis, conventional purifying techniques such as salting-out, extraction with solvents, distillation, column chromatography, liquid chromatography, electrophoresis, recrystallization, etc. are optionally combined so that the protein of the present invention can be obtained in a purified and isolated form. When the protein obtained as such is a free compound, it may be converted to a suitable salt by known methods while, when it is obtained as a salt, the salt may be converted to a free compound or other salt compounds by known methods.

Furthermore, the product may be manufactured by culturing a transformant (transfectant) containing a DNA coding for said protein or a fragment thereof.

The G protein coupled receptor protein-encoding DNA of the present invention may be any coding DNA as long as it contains a nucleotide sequence coding for a G protein coupled receptor protein which contains the amino acid sequence having SEQ ID NO: 1 or SEQ ID NO: 2 or a substantial equivalent thereof and/or which has an activity substantially equivalent to the amino acid sequence having SEQ ID NO: 1 or SEQ ID NO: 2.

The DNA (or its segment) of the present invention may be any one of a genome DNA, a genome DNA library, a tissue and cell-derived cDNA, a tissue and cell-derived cDNA library and a synthetic DNA. The vector used for the library may include bacteriophage, plasmid, cosmid, phagemid, etc. The DNA segment can be further amplified directly by the reverse transcriptase polymerase chain reaction (hereinafter briefly referred to as "RT-PCR") using mRNA fractions prepared from tissues and cells.

In one embodiment, the DNA segment coding for the G protein coupled receptor protein may be any coding DNA as long as it contains a nucleotide sequence coding for a rabbit gastropyrolic part smooth muscle-derived G protein coupled receptor protein which contains an amino acid sequence having the amino acid sequence of SEQ ID NO: 1 or SEQ ID NO: 2 or an substantial equivalent thereof and/or which has an activity substantially equivalent to the amino acid sequence having SEQ ID NO: 1 or SEQ ID NO: 2. Examples of a DNA segment coding for the rabbit gastropyrolic part smooth muscle-derived G protein coupled receptor protein comprising the amino acid sequence of SEQ ID NO: 1 includes DNA having a nucleotide sequence represented by SEQ ID NO: 3, etc. Examples of a DNA segment coding for the rabbit gastropyrolic part smooth muscle-derived G protein coupled receptor protein comprising the amino acid sequence of SEQ ID NO: 2 includes DNA having a nucleotide sequence represented by SEQ ID NO: 4, etc.

The DNA completely coding for the G protein coupled receptor protein of the present invention can be cloned by (a) carrying out PCR amplification using a synthetic DNA primer having a partial nucleotide sequence (nucleotide fragment) of the G protein coupled receptor protein; or (b) effecting the selection of a DNA constructed in a suitable vector, based on hybridization with a labeled DNA fragment having part or all of the region encoding a G protein coupled receptor protein or a labeled synthetic DNA having part or all of the coding region thereof. The hybridization is carried out according to methods such as disclosed in, for example, Molecular Cloning, 2nd Ed., J. Sambrook et al., Cold Spring Harbor Lab. Press, 1989. When a commercial DNA library is used, the hybridization is carried out according to the protocols or manuals attached thereto.

The cloned G protein coupled receptor protein-encoding DNA of the present invention can be used as it is, or it can be used, as desired, after modifications including digestion with a restriction enzyme or addition of a linker or adapter, etc. depending upon the objective. The DNA may have an initiation codon, ATG, on the 5' terminal side and a termination codon, TAA, TGA or TAG, on the 3' terminal side. These initiation and termination codons can be inserted by ligation using a suitable synthetic DNA adapter.

A vector containing the G protein coupled receptor protein-encoding DNA (for example, an expression vector for the G protein coupled receptor protein, etc.) can be produced by, for example, (a) cutting out a target DNA fragment from the G protein coupled receptor protein-encoding DNA of the present invention and (b) ligating the target DNA fragment with the downstream site of a promoter in a suitable expression vector (for example, an expression plasmid compatible with the G protein coupled receptor protein-encoding DNA, etc.).

The vector may include plasmids derived from Escherichia coli (e.g., pBR322, pBR325, pUC12, pUC13, etc.), plasmids derived from Bacillus subtilis (e.g., pUB110, pTP5, pC194, etc.), plasmids derived from yeasts (e.g., pSH19, pSH15, etc.), bacteriophages such as $\lambda$-phage, and animal virus such as retrovirus, vaccinia virus and baculovirus.

According to the present invention, any promoter can be used as long as it is compatible with the host cell which is used for expressing a gene. When the host for the transformation is E. coli, the promoters are preferably trp promoters, lac promoters, recA promoters, $\lambda_{PL}$ promoters, lpp promoters, etc. When the host for the transformation is Bacillus, the promoters are preferably SPO1 promoters, SPO2 promoters, penP promoters, etc. When the host is a yeast, the promoters are preferably PHO5 promoters, PGK promoters, GAP promoters, ADH promoters, etc. When the host is an animal cell, the promoters include SV40-derived promoters, retrovirus promoters, metallothionein promoters, heat shock promoters, cytomegalovirus (CMV) promoters, SR$\alpha$ promoters, etc. An enhancer can be effectively utilized for expression.

As required, furthermore, a host-compatible signal sequence is added to the N-terminal side of the G protein coupled receptor protein. When the host is E. coli, the utilizable signal sequences may include alkaline phosphatase signal sequences, OmpA signal sequences, etc. When the host is Bacillus, they may include $\alpha$-amylase signal sequences, subtilisin signal sequences, etc. When the host is a yeast, they may include mating factor $\alpha$ signal sequences, invertase signal sequences, etc. When the host is an animal cell, they may include insulin signal sequences, $\alpha$-interferon signal sequences, antibody molecule signal sequences, etc.

A transformant or transfectant is produced by using the vector thus constructed, which carries the G protein coupled receptor protein-encoding DNA of the present invention. The host may be, for example, Escherichia microorganisms, Bacillus microorganisms, yeasts, insect cells, animal cells, etc. Examples of the Escherichia and Bacillus microorganisms include Escherichia coli K12 · DH1 [Proc. Natl. Acad. Sci. USA, Vol. 60, 160 (1968)], JM103 [Nucleic Acids Research, Vol. 9, 309 (1981)], JA221 [Journal of Molecular Biology, Vol. 120, 517 (1978)], HB101 [Journal of Molecular Biology, Vol. 41, 459 (1969)], C600 [Genetics, Vol. 39, 440 (1954)], etc. Examples of the Bacillus microorganism are, for example, Bacillus subtilis MI114 [Gene, Vol. 24, 255 (1983)], 207-21 [Journal of Biochemistry, Vol. 95, 87 (1984)], etc. The yeast may be, for example, Saccharomyces cerevisiae AH22, AH22R⁻, NA87-11A, DKD-5D, 20B-12, etc. The insect may include a silkworm (Bombyx mori larva), [Maeda et al, Nature, Vol. 315, 592 (1985)] etc. The host animal cell may be, for example, monkey-derived cell line, COS-7, Vero, Chinese hamster ovary cell line (CHO cell), DHFR gene-deficient Chinese hamster cell line (dhfr CHO cell), mouse L cell, mouse myeloma cell, human FL, etc.

Depending on the host cell used, transformation is done using standard techniques appropriate to such cells. Transformation of Escherichia microorganisms can be carried out in accordance with methods as disclosed in, for example, Proc. Natl. Acad. Sci. USA, Vol. 69, 2110 (1972), Gene, Vol. 17, 107 (1982), etc. Transformation of Bacillus microorganisms can be carried out in accordance with methods as disclosed in, for example, Molecular & General Genetics, Vol. 168, 111 (1979), etc. Transformation of the yeast can be carried out in accordance with methods as disclosed in, for example, Proc. Natl. Acad. Sci. USA, Vol. 75, 1929 (1978), etc. The insect cells can be transformed in accordance with methods as disclosed in, for example, Bio/Technology, 6, 47-55, 1988. The animal cells can be transformed by methods as disclosed in, for example, Virology, Vol. 52, 456, 1973, etc. The transformants or transfectants wherein the expression vector carrying a G protein coupled receptor protein-encoding DNA harbors are produced according to the aforementioned techniques.

Cultivation of the transformant (transfectant) in which the host is Escherichia or Bacillus microorganism can be carried out suitably in a liquid culture medium. The culture medium may contains carbon sources, nitrogen sources, minerals, etc. necessary for growing the transformant. The carbon source may include glucose, dextrin, soluble starch, sucrose, etc. The nitrogen source may include organic or inorganic substances such as ammonium salts, nitrates, corn steep liquor, peptone, casein, meat extracts, bean-cakes, potato extracts, etc. Examples of the minerals may include calcium chloride, sodium dihydrogen phosphate, magnesium chloride, etc. It is further allowable to add yeasts, vitamins, growth-promoting factors, etc. It is desired that the culture medium is pH from about 5 to about 8.

The Escherichia microorganism culture medium is preferably an M9 medium containing, for example, glucose and casamino acid (Miller, Journal of Experiments in Molecular Genetics), 431-433, Cold Spring Harbor Laboratory, New York, 1972. Depending on necessity, the medium may be supplemented with drugs such as 3$\beta$-indolyl acrylic acid in order to improve efficiency of the promoter. In the case of an Escherichia host, the cultivation is carried out usually at about 15 to 43 °C for about 3 to 24 hours. As required, aeration and stirring may be applied. In the case of a Bacillus host, the cultivation is carried out usually at about 30 to 40 °C for about 6 to 24 hours. As required, aeration and stirring may be also applied. In the case of the transformant in which the host is a yeast, the culture medium used may include, for example, a Burkholder minimum medium [Bostian, K.L. et al., Proc. Natl. Acad. Sci. USA, Vol. 77, 4505 (1980)], an SD medium containing 0.5% casamino acid [Bitter, G.A. et al., Proc. Natl. Acad. Sci. USA, Vol. 81, 5330 (1984)], etc. It is preferable that the pH of the culture medium is adjusted to be from about 5 to about 8. The cultivation is carried out usually at about 20 to 35 °C for about 24 to 72 hours. As required, aeration and stirring may be applied.

In the case of the transformant in which the host is an insect, the culture medium used may include those obtained by suitably adding additives such as passivated (or immobilized) 10% bovine serum and the like to the Grace's insect medium (Grace, T.C.C., Nature, 195, 788 (1962)). It is preferable that the pH of the culture medium is adjusted to be about 6.2 to 6.4. The cultivation is usually carried out at about 27 °C for about 3 to 5 days. As desired, aeration and stirring may be applied. In case of the transformant in which the host is an animal cell, the culture medium used may include MEM medium [Science, Vol. 122, 501 (1952)], DMEM medium [Virology, Vol. 8, 396 (1959)], RPMI 1640 medium [Journal of the American Medical Association, Vol. 199, 519 (1967)], 199 medium [Proceedings of the Society for the Biological Medicine, Vol. 73, 1 (1950)], etc. which are containing, for example, about 5 to 20% of fetal calf serum. It is preferable that the pH is from about 6 to about 8. The cultivation is usually carried out at about 30 to 40 °C for about 15 to 60 hours. As required, medium exchange, aeration and stirring may be applied.

Separation and purification of the G protein coupled receptor protein from the above-mentioned cultures can be carried out according to methods described herein below.

To extract G protein coupled receptor proteins from the cultured microorganisms or cells, the microorganisms or cells are collected by known methods after the cultivation, suspended in a suitable buffer solution, disrupted by ultrasonic waves, lysozyme and/or freezing and thawing, etc. and, then, a crude extract of the G protein coupled receptor protein is obtained by centrifugation or filtration. Other conventional extracting or isolating methods can be applied. The buffer solution may contain a protein-denaturing agent such as urea or guanidine hydrochloride or a surfactant such as Triton X-100 (registered trademark, hereinafter often referred to as "TM").

In the case where G protein coupled receptor proteins are secreted into culture media, supernatant liquids are separated from the microorganisms or cells after the cultivation is finished and the resulting supernatant liquid is collected by widely known methods. The culture supernatant liquid and extract containing G protein coupled receptor proteins can be purified by suitable combinations of widely known methods for separation, isolation and purification. The widely known methods of separation, isolation and purification may include methods which utilizes solubility, such as salting out or sedimentation with solvents methods which utilizes chiefly a difference in the molecular size or weight, such as dialysis, ultrafiltration, gel filtration and SDS-polyacrylamide gel electrophoresis, methods utilizing a difference in the electric charge, such as ion-exchange chromatography, methods utilizing specific affinity such as affinity chromatography, methods utilizing a difference in the hydrophobic property, such as inverse-phase high-performance liquid chromatography, and methods utilizing a difference in the isoelectric point such as isoelectric electrophoresis, etc.

In cases where the G protein coupled receptor protein thus obtained is in a free form, the free protein can be converted into a salt thereof by known methods or method analogous thereto. In case where the G protein coupled receptor protein thus obtained is in a salt form vice versa, the protein salt can be converted into a free form or into any other salt thereof by known methods or method analogous thereto.

The G protein coupled receptor protein produced by the transformant can be arbitrarily modified or a polypeptide can be partly removed therefrom, by the action of a suitable protein-modifying enzyme before or after the purification. The protein-modifying enzyme may include trypsin, chymotrypsin, arginyl endopeptidase, protein kinase, glycosidase, etc. The activity of the G protein coupled receptor protein thus formed can be measured by experimenting the coupling (or binding) with a ligand or by enzyme immunoassays (enzyme linked immunoassays) using specific antibodies.

The G protein coupled receptor protein, the partial peptide (fragment) thereof and the G protein coupled receptor protein-encoding DNA of the present invention can be used for:

① determining a ligand to the G protein coupled receptor protein of the present invention,
② obtaining an antibody and an antiserum,
③ constructing a system for expressing a recombinant receptor protein,
④ developing a receptor-binding assay system using the above developing system and screening pharmaceutical candidate compounds,
⑤ designing drugs based upon comparison with ligands and receptors which have a similar or analogous structure,
⑥ preparing a probe for the analysis of genes and preparing a PCR primer,
⑦ gene manipulation therapy,
⑧ producing a transgenic animal (for example, transgenic mouse, etc.),
⑧ producing a model animal suffering from diseases caused by gene deficiency, etc.

In particular, it is possible to screen a G protein coupled receptor agonist or antagonist specific to a warm-blooded animal such as human being by a receptor-binding assay system which uses a system for expressing a recombinant G protein coupled receptor protein of the present invention. The agonist or antagonist thus screened or characterized permits various applications including prevention and/or therapy of a variety of diseases.

Described below are uses of G protein coupled receptor proteins, partial peptides thereof (peptide fragments thereof), G protein coupled receptor protein-encoding DNAs and antibodies against-the G protein coupled receptor protein according to the present invention.

As hereunder described, more detailed description will be made on the usefulness of the G protein coupled receptor protein-encoding DNAs according to the present invention, the G protein coupled receptor proteins encoded by said DNA, partial peptides thereof (including peptide fragments or segments thereof) or salts thereof (hereinafter, those including their salts, will be referred to as the "G protein coupled receptor protein"), cells or cell membrane fractions thereof each containing the recombinant type G protein coupled receptor protein, etc. Their various applications are also disclosed herein below.

(1) Method for Determining a Ligand to the G Protein Coupled Receptor Protein

The G protein coupled receptor protein, the fragment thereof or a salt thereof is useful as a reagent for investigating or determining a ligand to said G protein coupled receptor protein.

According to the present invention, methods for determining a ligand to the G protein coupled receptor protein which comprises contacting the G protein coupled receptor protein or the peptide fragment thereof with the compound to be tested are provided.

The compound to be tested may include not only known ligands such as angiotensins, bombesins, canavinoids, cholecystokinins, glutamine, serotonin, melatonins, neuropeptides Y, opioids, purine, vasopressins, oxytocins, VIP (vasoactive intestinal and related peptides), somatostatins, dopamine, motilins, amylins, bradykinins, CGRP (calcitonin gene related peptides), adrenomedullins, leukotrienes, pancreastatins, prostaglandins, thromboxanes, adenosine, adrenaline, $\alpha$- and $\beta$-chemokines (IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelins, enterogastrins, histamine, neurotensins, TRH, pancreatic polypeptides, galanin, modified derivatives thereof, analogues thereof, family members thereof and the like but also tissue extracts, cell culture supernatants, etc. of warm-blooded animals (such as guinea pig, rabbits, mice, rats, swines, cattle, sheep, monkeys and human being), etc. For example, said tissue extract, said cell culture supernatant, etc. is added to the G protein coupled receptor protein for measurement of the cell stimulating activity, etc. and fractionated by relying on the measurements whereupon a single ligand can be finally determined and obtained.

In one specific embodiment of the present invention, said method for determining the ligand includes a method for determining whether a sample (including a compound or a salt thereof) is capable of stimulating a target cell which comprises binding said compound with the G protein coupled receptor protein either in the presence of the G protein coupled receptor protein, the fragment thereof or a salt thereof, or in a receptor binding assay system in which the expression system for the recombinant type receptor protein is constructed and used; and measuring the receptor-mediated cell stimulating activity, etc. Examples of said cell stimulating activities that can be measured include promoting or inhibiting biological responses, e.g. liberation of arachidonic acid, liberation of acetylcholine, liberation of endocellular $Ca^{2+}$, production of endocellular cAMP, production of endocellular cGMP, production of inositol phosphate, changes in the cell membrane potential, phosphorylation of endocellular protein, activation of c-fos, decrease in pH, etc. Examples of said compound or its salt capable of stimulating the cell via binding with the G protein coupled receptor protein include peptides, proteins, nonpeptidic compounds, synthetic compounds, fermented products, etc.

In said method for determining the ligand, the characteristic feature of this screening is that when the G protein coupled receptor protein or the peptide fragment thereof is contacted with the test compound, for example, the binding amount, the cell stimulating activity, etc. of the test compound to the G protein coupled receptor protein or the peptide fragment thereof is measured.

In more specific embodiments of the present invention, said methods for screening and identifying a ligand includes:

① a method of screening for a ligand to a G protein coupled receptor protein, which comprises contacting a labeled test compound with a G protein coupled receptor protein or a peptide fragment thereof, and measuring the amount of the labeled test compound binding with said protein or salt thereof or with said fragment or salt thereof;

② a method of screening for a ligand to a G protein coupled receptor protein, which comprises contacting a labeled test compound with cells containing the G protein coupled receptor protein or the membrane fraction of said cell, and measuring the amount of the labeled test compound binding with said cells or said cell fraction;

③ a method of screening for a ligand to a G protein coupled receptor protein, which comprises contacting a labeled test compound with the G protein coupled receptor protein expressed on cell membranes by culturing transformants carrying the G protein coupled receptor protein-encoding DNA, and measuring the amount of the labeled test compound binding with said G protein coupled receptor protein;

④ a method of screening for a ligand to a G protein coupled receptor protein, which comprises contacting a test compound with cells containing the G protein coupled receptor protein, and measuring the cell stimulating activity (e.g. promoting or inhibiting activity on biological responses such as liberation of arachidonic acid, liberation of acetylcholine, liberation of endocellular $Ca^{2+}$, production of endocellular cAMP, production of endocellular cGMP, production of inositol phosphate, changes in the cell membrane potential, phosphorylation of endocellular protein, activation of c-fos, lowering in pH, etc.) via the G protein coupled receptor protein; and

⑤ a method of screening for a ligand to the G protein coupled receptor protein, which comprises contacting a test compound with the G protein coupled receptor protein expressed on the cell membrane by culturing transformants carrying the G protein coupled receptor protein-encoding DNA, and measuring at least one cell stimulating activity, e.g., an activity for promoting or inhibiting physiological responses such as liberation of arachidonic acid, liberation of acetylcholine, liberation of endocellular $Ca^{2+}$, production of endocellular cAMP, production of endocellular cGMP, production of inositol phosphate, changes in the cell membrane potential, phosphorylation of endocellular protein, activation of c-fos, lowering in pH, activation of G protein, cell promulgation, etc.) via the G protein coupled receptor protein.

Described below are specific illustrations of the method for screening and identifying ligands according to the present invention which are provided only for illustrative purposes.

First, the G protein coupled receptor protein used for the method for determining the ligand may include any material so far as it contains a G protein coupled receptor protein, a partial peptide thereof (e.g., a peptide fragment as defined above) or a salt thereof although it is preferable to express large amounts of the G protein coupled receptor proteins in animal cells.

In the manufacture of the G protein coupled receptor protein, the above-mentioned method can be used and carried out by expressing said protein encoding DNA in mammalian cells or in insect cells. With respect to the DNA fragment coding for a particular region such as an extracellular epitope, the extracellular domains, etc., complementary DNA may be used although the method of expression is not limited thereto. For example, gene fragments or synthetic DNA may be used as well.

In order to introduce the G protein coupled receptor protein-encoding DNA fragment into host animal cells and to express it efficiently, it is preferred that said DNA fragment is incorporated into the downstream site of polyhedron promoters derived from nuclear polyhedrosis virus belonging to baculovirus, promoters derived from SV40, promoters derived from retrovirus, metallothionein promoters, human heat shock promoters, cytomegalovirus promoters, SRα promoters, etc. Examinations of the quantity and the quality of the expressed receptor can be carried out by methods per se known to those of skill in the art or methods similar thereto based upon the present disclosure. For example, they may be conducted by methods described in publications such as Nambi, P. et al: The Journal of Biochemical Society, vol.267, pages 19555-19559 (1992).

Accordingly, with respect to the determination of the ligand, the material containing a G protein coupled receptor protein or peptide fragment thereof may include products containing G protein coupled receptor proteins which are purified by methods per se known to those of skill in the art or methods similar thereto, peptide fragments of said G protein coupled receptor protein, cells containing said G protein coupled receptor protein, membrane fractions of the cell containing said protein, etc.

When the G protein coupled receptor protein-containing cell is used in the determining method of the ligand, said cell may be immobilized with binding agents including glutaraldehyde, formalin, etc. The immobilization may be carried out by methods per se known to those of skill in the art or methods similar thereto.

The G protein coupled receptor protein-containing cells are host cells which express the G protein coupled receptor protein. Preferably the cell is a stably transformed cell line. However, transiently transformed cells can also be used. Examples of said host cells are microorganisms such as Escherichia coli, Bacillus subtilis, yeasts, insect cells, animal cells, etc.

The cell membrane fraction is a cell membrane-rich fraction which is prepared by methods per se known to those of skill in the art or methods similar thereto after disruption of cells. Examples of cell disruption may include a method for squeezing cells using a Potter-Elvejem homogenizer, a disruption by a Waring blender or a Polytron (manufactured by Kinematica), a disruption by ultrasonic waves, a disruption via blowing out cells from small nozzles together with applying a pressure using a French press or the like, etc. In the fractionation of the cell membrane, a fractionation method by means of centrifugal force such as a fractional centrifugal separation and a density gradient centrifugal separation is mainly used. For example, disrupted cellular liquid is centrifuged at a low speed (500 rpm to 3,000 rpm) for a short period (usually, from about one to ten minutes), the supernatant liquid is further centrifuged at a high speed (15,000 rpm to 30,000 rpm) usually for 30 minutes to two hours and the resulting precipitate is used as a membrane fraction. Said membrane fraction contains a lot of the expressed G protein coupled receptor protein and a lot of membrane components such as phospholipids and membrane proteins derived from the cells.

The amount of the G protein coupled receptor protein in the membrane fraction cell containing said G protein coupled receptor protein is preferably $10^3$ to $10^8$ molecules per cell or, more preferably, $10^5$ to $10^7$ molecules per cell. Incidentally, the greater the expressed amount, the higher the ligand binding activity (specific activity) per membrane fraction whereby the construction of a highly sensitive screening system becomes possible and, moreover, it permits measurement of a large amount of samples within the same lot.

In conducting the above-mentioned methods ① to ③ wherein ligands capable of binding with the G protein coupled receptor protein are determined, a suitable G protein coupled receptor fraction and a labeled test compound are necessary. The G protein coupled receptor fraction is preferably a naturally occurring (natural type) G protein coupled receptor, a recombinant type G protein coupled receptor having the activity equivalent to that of the natural type. Here, the term "activity equivalent to" means the equivalent ligand binding activity, etc. as discussed above.

Suitable examples of the labeled test compound are angiotensin, bombesin, canavinoid, cholecystokinin, glutamine, serotonin, melatonin, neuropeptide Y, opioid, purine, vasopressin, oxytocin, VIP (vasoactive intestinal and related peptides), somatostatin, dopamine, motilin, amylin, bradykinin, CGRP (calcitonin gene related peptides), adrenomedullin, leukotriene, pancreastatin, prostaglandin, thromboxane, adenosine, adrenaline, $\alpha$- and $\beta$-chemokine (IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptides, galanin, an analogue derivative thereof, etc. which are labeled with [$^3$H], [$^{125}$I], [$^{14}$C], [$^{35}$S], etc.

Specifically, the determination of ligands capable of binding with G protein coupled receptor proteins is carried out as follows:

First, cells or cell membrane fractions containing the G protein coupled receptor protein are suspended in a buffer suitable for the assay to prepare the receptor sample for conducting the method of determining the ligand binding with the G protein coupled receptor protein. The buffer may include any buffer such as Tris-HCl buffer or phosphate buffer with pH 4-10 (preferably, pH 6-8), etc., as long as it does not inhibit the binding of the ligand with the receptor. In addition, surface-active agents such as CHAPS, Tween 80™ (Kao-Atlas, Japan), digitonin, deoxycholate, etc. and various proteins such as bovine serum albumin (BSA), gelatin, milk derivatives, etc. may be added to the buffer with an object of decreasing the non-specific binding. Further, a protease inhibitor such as PMSF, leupeptin, E-64 (manufactured by Peptide Laboratory), pepstatin, etc. may be added with an object of inhibiting the decomposition of the receptor and the ligand by protease. A test compound labeled with a predetermined (or certain) amount (5,000 cpm to 500,000 cpm) of [$^3$H], [$^{125}$I], [$^{14}$C], [$^{35}$S], etc. coexists in 0.01 ml to 10 ml of said receptor solution.

In order to know the non-specific binding amount (NSB), a reaction tube to which a great excessive amount of the unlabeled test compound is added is prepared as well. The reaction is carried out at 0-50° C (preferably at 4-37° C) for 20 minutes to 24 hours (preferably 30 minutes to three hours). After the reaction, it is filtered through a glass fiber filter or the like, washed with a suitable amount of the same buffer and the radioactivity remaining in the glass fiber filter is measured by means of a liquid scintillation counter or a gamma-counter. The test compound in which the count (B - NSB) obtained by subtracting the non-specific binding amount (NSB) from the total binding amount (B) is more than 0 cpm is identified as a ligand to the G protein coupled receptor protein of the present invention.

In conducting the above-mentioned methods ④ to ⑤ wherein ligands capable of binding with the G protein coupled receptor protein are determined, the cell stimulating activity (e.g. the liberation of arachidonic acid, the liberation of acetylcholine, endocellular Ca$^{2+}$ liberation, endocellular cAMP production, the production of inositol phosphate, changes in the cell membrane potential, the phosphorylation of endocellular protein, the activation of c-fos, lowering of pH, the activation of G protein, cell promulgation, etc.) mediated by the G protein coupled receptor protein may be measured by known methods or by the use of commercially available measuring kits. To be more specific, G protein coupled receptor protein-containing cells are at first cultured in a multi-well plate or the like.

In conducting the determination of ligand, it is substituted with a fresh medium or a suitable buffer which does not show toxicity to the cells in advance of the experiment, and incubated under appropriate conditions and for sufficient time after adding a test compound, etc. thereto. Then, the cells are extracted or the supernatant liquid is recovered and the resulting product is determined by each of the methods. When it is difficult to identify the production of the substance (e.g. arachidonic acid, etc.) which is to be an index for the cell stimulating activity due to the decomposing enzyme contained in the cell, an assay may be carried out by adding an inhibitor against said decomposing enzyme. With respect to an activity such as an inhibitory action against cAMP production, it may be detected as an inhibitory action against the production of the cells whose fundamental production is increased by forskolin or the like.

The kit used for the method of determining the ligand binding with the G protein coupled receptor protein includes a G protein coupled receptor protein or a fragment thereof, cells containing the G protein coupled receptor protein, a membrane fraction from the cells containing the G protein coupled receptor protein, etc.

Examples of the kit for determining the ligand are as follows:

1. Reagent for Determining the Ligand.

① Buffer for Measurement and Buffer for Washing.

The buffering product wherein 0.05% of bovine serum albumin (manufactured by Sigma) is added to Hanks' Balanced Salt Solution (manufactured by Gibco).

This product may be sterilized by filtration through a membrane filter with a 0.45 μm pore size, and stored at 4°C or may be formulated upon use.

② G Protein Coupled Receptor Protein Sample.

CHO cells in which G protein coupled receptor proteins are expressed are subcultured at the rate of $5 \times 10^5$ cells/well in a 12-well plate and cultured at 37°C in a humidified 5% $CO_2$/95% air atmosphere for two days to prepare the sample.

③ Labeled Test Compound.

The compound which is labeled with commercially available [$^3$H], [$^{125}$I], [$^{14}$C], [$^{35}$S], etc. or labeled with a suitable method.

The product in a state of an aqueous solution is stored at 4°C or at -20° C and, upon use, diluted to 1 μM with a buffer for the measurement. In the case of a test compound which is barely soluble in water, it may be dissolved in an organic solvent such as dimethylformamide, DMSO, methanol and the like.

④ Unlabeled Test Compound.

The same compound as the labeled one is prepared in a concentration of 100 to 1,000-fold concentrated state.

2. Method of Measurement.

① G protein coupled receptor protein-expressing CHO cells cultured in a 12-well tissue culture plate are washed twice with 1 ml of buffer for the measurement and then 490 μl of buffer for the measurement is added to each well.
② Five μl of the labeled test compound is added and the mixture is made to react at room temperature for one hour. For measuring the nonspecific binding amount, 5 μl of the unlabeled test compound is added.
③ The reaction solution is removed from each well, which is washed with 1 ml of a buffer for the measurement three times. The labeled test compound which is binding with the cells is dissolved in 0.2N NaOH-1% SDS and mixed with 4 ml of a liquid scintillator A (manufactured by Wako Pure Chemical, Japan).
④ Radioactivity is measured using a liquid scintillation counter such as one manufactured by Beckmann.

The ligand which can bind with the G protein coupled receptor protein include substances occurring or existing, for example, in brain, pituitary gland, pancreas, stomach, etc. Examples of the ligand are angiotensin, bombesin, canavinoid, cholecystokinin, glutamine, serotonin, melatonin, neuropeptide Y, opioid, purine, vasopressin, oxytocin, VIP (vasoactive intestinal and related peptide), somatostatin, dopamine, motilin, amylin, bradykinin, CGRP (calcitonin gene related peptide), adrenomedullin, leukotriene, pancreastatin, prostaglandin, thromboxane, thromboxatin, adenosine, adrenaline, $\alpha$- and $\beta$-chemokine (IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptide, galanin, modified derivatives thereof, analogues thereof, etc.

(2) Prophylactic and Therapeutic Agent for G Protein Coupled Receptor Protein Deficiency Diseases

If a ligand to the G protein coupled receptor protein is revealed via the aforementioned method (1), the G protein coupled receptor protein-encoding DNA can be used as a prophylactic and/or therapeutic agent for treating said G protein coupled receptor protein deficiency diseases depending upon the action that said ligand exerts.
For example, when there is a patient for whom the physiological action of the ligand cannot be expected because of a decrease in the G protein coupled receptor protein in vivo, the amount of the G protein coupled receptor protein in the brain cells of said patient can be increased whereby the action of the ligand can be fully achieved by:

(a) administering the G protein coupled receptor protein-encoding DNA to the patient to express it; or
(b) inserting the G protein coupled receptor protein-encoding DNA into brain cells or the like to express it, followed by transplanting said brain cells or the like to said patient. Accordingly, the G protein coupled receptor protein-encoding DNA can be used as a safe and less toxic preventive and therapeutic agent for the G protein coupled receptor protein deficiency diseases.

When the G protein coupled receptor protein-encoding DNA is used as the above-mentioned agent, said DNA may be used alone or after inserting it into a suitable vector such as retrovirus vector, adenovirus vector, adenovirus-associated virus vector, etc. followed by subjecting the product vector to a conventional means. Thus, it may be administered orally parenterally, by inhalation spray, rectally, or topically as pharmaceutical compositions or formulations. It may also be administered by use of catheters. Oral formulations include tablets (sugar-coated if necessary), capsules, elixirs, microcapsules, etc. Parenteral formulations include injections such as an aseptic solution or a suspension in water or in other pharmaceutically acceptable liquid. For example, the DNA of the present invention is admixed in a unit dose form which is required for preparing generally approved pharmaceutical preparations together with a physiologically acceptable carriers, flavoring agents, adjuvants, excipients, diluents, fillers, vehicles, antiseptics, stabilizers, binders, etc. whereupon the preparation can be manufactured. The amount of the effective component in those preparations is to be in such an extent that the suitable dose within an indicated range is achieved.

Examples of the additives which can be admixed in the tablets, capsules, etc. are binders such as gelatin, corn starch, tragacanth and gum arabicum; fillers such as crystalline cellulose; swelling agents such as corn starch, gelatin and alginic acid; lubricating agents such as magnesium stearate; sweetening agents such as sucrose, lactose and saccharine; and flavoring agents such as pepper mint, akamono oil and cherry. When the unit dose form of the preparation is a capsule, a liquid carrier such as fat/oil may be further added in addition of the above-mentioned types of materials. The aseptic composition for injection may be formulated by conventional practices for the preparations such as that the active substance in a vehicle such as water for injection is dissolved or suspended in naturally occurring plant oil such as sesame oil and palm oil.

These pharmaceutical compounds are prepared under aseptic conditions using accepted pharmaceutical criteia. It should be substantially free of pyrogens and endotoxins.

Examples of an aqueous liquid for injection are a physiological saline solution and isotonic solutions containing glucose and other auxiliary agents (e.g. D-sorbitol, D-mannitol, sodium chloride, etc.) wherein a suitable auxiliary solubilizers such as alcohol (e.g. ethanol, etc.), polyalcohol (e.g. propylene glycol polyethylene glycol, etc.), nonionic surface-active agent (e.g. Polysorbate 80™, HCO-50, etc.), etc. may be jointly used. Examples of an oily liquid include sesame oil, soybean oil, etc. wherein benzyl benzoate, benzyl alcohol, etc. may be jointly used as auxiliary solubilizers. In addition, buffers (e.g. phosphate buffer, sodium acetate buffer, etc.), analgesic agents (e.g. benzalkonium chloride, procaine hydrochloride, etc.), stabilizers (e.g. human serum albumin, polyethylene glycol, etc.), stabilizers (e.g. benzyl alcohol phenol, etc.), antioxidants, etc. may be admixed therewith too. The prepared injection solution is filled in suitable ampoules. The preparation prepared as such is safe and less toxic and, therefore, it can be administered to warm-blooded animals (e.g., rat, rabbit, sheep, swine, cattle, cat, dog, monkey, human beings, etc.).

Specific dose levels of said DNA may vary depending upon a variety of factors including the activity of drugs employed, the age, body weight, general health, sex, diet, time of administration, route of administration, drug combination, and the severity of the symptom. In the case of oral administration, it is usually about 0.1-100 mg, preferably about 1.0-50 mg or, more preferably, about 1.0-20 mg per day for adults (as 60 kg). When it is administered parenterally, its dose at a time may vary depending upon the object (patient) to be administered, organs to be administered, symptoms, administering methods, etc. but, in the case of injections, it is usually convenient to give by an intravenous route in an amount of about 0.01-30 mg, preferably about 0.1-20 mg or, more preferably, about 0.1-10 mg per day to adults (as 60 kg). In the case of other animals, the dose calculated for 60 kg may be administered as well.

(3) Quantitative Determination of Ligand to the G Protein Coupled Receptor Protein of the Present Invention.

The G protein coupled receptor protein or a fragment thereof that has a binding property for a ligand are capable of determining quantitatively an amount of ligands in vivo with good sensitivity.

This quantitative determination may be carried out by, for example, combining with a competitive analysis. Thus, a sample to be determined is contacted with G protein coupled receptor proteins or peptide fragments thereof so that the ligand concentration in said sample can be determined. In one embodiment of the quantitative determination, the protocols described in the following ① and ② or methods similar thereto may be used:

① Hiroshi Irie (ed): "Radioimmunoassay" (Kodansha, Japan, 1974); and
② Hiroshi Irie (ed): "Radioimmunoassay, Second Series" (Kodansha, Japan, 1979).

(4) Screening of Compound Inhibiting the Binding of Ligand with the G Protein Coupled Receptor Protein of the Present Invention.

G Protein coupled receptor proteins or fragments thereof can be used. Alternatively, expression systems for recombinant type G Protein coupled receptor proteins or fragments thereof are constructed and receptor binding assay systems using said expression system are used. In these assay systems, it is possible to screen compounds (e.g. peptides, proteins, nonpeptidic compounds, synthetic compounds, fermented products, cell extracts, animal tissue extracts, etc.) or salts thereof which inhibits the binding of a ligand with the G protein coupled receptor protein. Such a compound includes a compound exhibiting a G protein coupled receptor-mediated cell stimulating activity (e.g. activity of promoting or activity of inhibiting physiological reactions including liberation of arachidonic acid, liberation of acetylcholine, endocellular $Ca^{2+}$ liberation, endocellular cAMP production, endocellular cGMP production, production of inositol phosphate, changes in cell membrane potential, phosphorylation of endocellular proteins, activation of c-fos, lowering of pH, activation of G protein, cell promulgation, etc.) (so-called "G protein coupled receptor-agonist"), a compound free from such a cell stimulating activity (so-called "G protein coupled receptor-antagonist"), etc.

Thus, the present invention provides a method of screening for a compound which inhibits the binding of a ligand with a G protein coupled receptor protein or a salt thereof, characterized by comparing the following two cases:

(i) the case wherein the ligand is contacted with the G protein coupled receptor protein or salt thereof, or a fragment thereof or a salt thereof; and
(ii) the case wherein the ligand is contacted with a mixture of the G protein coupled receptor protein or salt thereof or the peptide fragment or salt thereof and said test compound.

In said screening method, one characteristic feature of the present invention resides in that the amount of the ligand bonded with said G protein coupled receptor protein or the fragment (partial peptide) thereof, the cell stimulating activity of the ligand, etc. are measured in both the case where (i) the ligand is contacted with G protein coupled receptor proteins or peptide fragments thereof and in the case where (ii) the ligand and the test compound are contacted with the G protein coupled receptor protein or the fragment thereof, respectively and then compared therebetween. This permits one to determine the dffect of the test compound.

In one more specific embodiment of the present invention, the following is provided:

① a method of screening for a compound or a salt thereof which inhibits the binding of a ligand with a G protein coupled receptor protein, characterized in that, when a labeled ligand is contacted with a G protein coupled receptor protein or a fragment thereof and when a labeled ligand and a test compound are contacted with a G protein coupled receptor protein or a fragment thereof, the amounts of the labeled ligand bonded with said protein or a fragment thereof or a salt thereof are measured and compared;

② a method of screening for a compound or a salt thereof which inhibits the binding of a ligand with a G protein coupled receptor protein, characterized in that, when a labeled ligand is contacted with cells containing G protein coupled receptor proteins or a membrane fraction of said cells and when a labeled ligand and a test compound are contacted with cells containing G protein coupled receptor proteins or a membrane fraction of said cells, the amounts of the labeled ligand binding with said protein or a fragment thereof or a salt thereof are measured and compared;

③ a method of screening for a compound or a salt thereof which inhibits the binding of a ligand with a G protein coupled receptor protein, characterized in that, when a labeled ligand is contacted with G protein coupled receptor proteins expressed on the cell membrane by culturing a transformant carrying a G protein coupled receptor protein-encoding DNA and when a labeled ligand and a test compound are contacted with G protein coupled receptor proteins expressed on the cell membrane by culturing a transformant carrying a G protein coupled receptor protein-encoding DNA, the amounts of the labeled ligand binding with said G protein coupled receptor protein are measured and compared;

④ a method of screening for a compound or a salt thereof which inhibits the binding of a ligand with a G protein coupled receptor protein, characterized in that, when a G protein coupled receptor protein-activating compound (e.g. a ligand to the G protein coupled receptor protein, etc.) is contacted with cells containing G protein coupled receptor proteins and when the G protein coupled receptor protein-activating compound and a test compound are contacted with cells containing G protein coupled receptor proteins, the resulting G protein coupled receptor protein-mediated cell stimulating activities (e.g. activities of promoting or activities of inhibiting physiological responses including liberation of arachidonic acid, liberation of acetylcholine, endocellular $Ca^{2+}$ liberation, endocellular cAMP production, endocellular cGMP production, production of inositol phosphate, changes in cell membrane potential, phosphorylation of endocellular proteins, activation of c-fos, lowering of pH, activation of G protein, cell promulgation, etc.) are measured and compared; and

⑤ a method of screening for a compound or a salt thereof which inhibits the binding of a ligand with a G protein coupled receptor protein, characterized in that, when a G protein coupled receptor protein-activating compound (e.g. a ligand to the G protein coupled receptor protein, etc.) is contacted with G protein coupled receptor proteins expressed on cell membranes by culturing transformants carrying G protein coupled receptor protein-encoding DNA and when a G protein coupled receptor protein-activating compound and a test compound are contacted with the G protein coupled receptor protein expressed on the cell membrane by culturing the transformant carrying the G protein coupled receptor protein-encoding DNA, the resulting G protein coupled receptor protein-mediated cell stimulating activities (activities of promoting or activities of inhibiting physiological responses such as liberation of arachidonic acid, liberation of acetylcholine, endocellular $Ca^{2+}$ liberation, endocellular cAMP production, endocellular cGMP production, production of inositol phosphate, changes in cell membrane potential, phosphorylation of endocellular proteins, activation of c-fos, lowering of pH, activation of G protein, and cell promulgation, etc.) are measured and compared.

Before the G protein coupled receptor protein of the present invention was obtained, the G protein coupled receptor agonist or antagonist had to be screened by, first, obtaining a candidate compound by using G protein coupled receptor protein-containing cells, tissues or cell membrane fractions derived from rat, rabbit or the like (primary screening) and, then, making sure whether the candidate compound really inhibits the binding between human G protein coupled receptor proteins and ligands (secondary screening). Other receptor proteins inevitably exist and when the cells, the tissues or the cell membrane fractions were used, they intrinsically make it difficult to screen agonists or antagonists to the desired receptor proteins. By using the present human-derived G protein coupled receptor protein, however, there is no need of effecting the primary screening, whereby it is possible to efficiently screen a compound that inhibits the binding between a ligand and a G protein coupled receptor. Additionally, it is possible to evaluate whether the compound that is screened is a G protein coupled receptor agonist or a G protein coupled receptor antagonist.

Specific explanations of the screening method will be given as hereunder.

First, with respect to the G protein coupled receptor protein used for the screening method of the present invention, any product may be used so far as it contains G protein coupled receptor proteins or fragments thereof although the use of a membrane fraction of mammalian organs is preferable. However, human organs can be extremely scarce and, accordingly, G protein coupled receptor proteins which are expressed in a large amount using a recombinant technique are suitable for the screening.

In the manufacture of the G protein coupled receptor protein, the above-mentioned method can be used and it may be carried out by expressing the DNA coding for said protein in mammalian cells or in insect cells. With respect to the DNA fragment coding for the target region, complementary DNA may be used although it is not limited thereto. Thus, for example, gene fragments or synthetic DNA may be used as well. One may also use mRNA under appropriate conditions.

In order to introduce the G protein coupled receptor protein-encoding DNA fragment into host animal cells and to express it efficiently, it is preferred that said DNA fragment is incorporated downstream of a promoter such as a polyhedron promoter of nuclear polyhedrosis virus belonging to baculovirus, a promoter derived from SV40, a promoter of retrovirus, a metallothionein promoter, a human heat shock promoter, a cytomegalovirus promoter, a SR$\alpha$ promoter, etc. Examinations of the quantity and the quality of expressed receptors can be carried out by known methods per se or modified methods substantially analogous thereto. For example, they may be conducted by the method described in publications such as Nambi, P. et al.: The Journal of Biochemical Society, vol.267, pages 19555-19559 (1992).

Accordingly, in the screening method, the substance containing a G protein coupled receptor protein or a fragment thereof may be a G protein coupled receptor protein which is purified by known methods per se or a G protein coupled receptor protein fragment (partial peptide) which is purified by known methods per se, or a cell containing said protein or a cell membrane fraction of the cell containing said protein, etc.

When the G protein coupled receptor protein-containing cells are used in the screening method, said cells may be immobilized with glutaraldehyde, formalin, etc. The immobilization may be carried out by known methods per se or modified methods substantially analogous thereto.

The G protein coupled receptor protein-containing cells are host cells expressing the G protein coupled receptor protein. Examples of said host cells may include Escherichia coli, Bacillus subtilis, yeasts, insect cells, animal cells such as CHO cell and COS cell, etc.

Cell membrane fractions are fractions which contain a lot of cell membranes prepared by known methods per se or modified methods substantially analogous thereto after disrupting or crushing the cells. Examples of disruptions of the cell may include methods by squeezing the cells with a Potter-Elvejem homogenizer, disrupting or crushing by a Waring blender or a Polytron (manufactured by Kinematica), disrupting or crushing by means of ultrasonic wave, disrupting by blowing out the cells from small nozzles together with applying a pressure with a French press or the like, etc. Fractionation of the cell membrane is carried out mainly by fractionation techniques by means of centrifugal force such as a fractional centrifugal separation and a density gradient centrifugal separation. For example, disrupted liquid of cells is centrifuged at a low speed (500 rpm to 3,000 rpm) for a short period (usually, from about one to ten minutes), the supernatant liquid is further centrifuged at a high speed (15,000 rpm to 30,000 rpm) usually for 30 minutes to two hours and the resulting precipitate is used as a membrane fraction. Said membrane fraction contains a lot of expressed G protein coupled receptor proteins and membrane components such as phospholipids and membrane proteins derived from the cells.

The amount of the G protein coupled receptor protein in the G protein coupled receptor protein-containing cell and in the cell membrane fraction obtained from the cell is preferably $10^3$ -$10^8$ molecules per cell or, more preferably, $10^5$ to $10^7$ molecules per cell. Incidentally, the more the expressed amount, the higher the ligand binding activity (specific activity) per membrane fraction whereby the construction of a highly sensitive screening system is possible and, moreover, permitting measurement of large amount of samples in the same lot.

In conducting the above-mentioned methods ① to ③ for screening the compound capable of inhibiting the binding of the ligand with the G protein coupled receptor protein, a suitable G protein coupled receptor fraction and a labeled ligand are necessary. With respect to the G protein coupled receptor fraction, it is preferred to use naturally occurring G protein coupled receptors (natural type G protein coupled receptors) or recombinant type G protein coupled receptor fractions with the activity equivalent to that of the natural type G protein coupled. Here the term "activity equivalent to" means the same ligand binding activity, or the substantially equivalent ligand binding activity. Preferably the activity is

EP 0 711 831 A2

at least about 75% of that of the wild type, more preferably at least about 85%, still more preferably at least about 90% and most preferably at least about 95%.

With respect to the labeled ligand, it is possible to use labeled ligands, labeled ligand analogized compounds, etc. For example, ligands labeled with [$^3$H], [$^{125}$I], [$^{14}$C], [$^{35}$S], etc. and other labeled substances may be utilized.

Specifically, G protein coupled receptor protein-containing cells or cell membrane fractions are first suspended in a buffer which is suitable for the determining method to prepare the receptor sample in conducting the screening for a compound which inhibits the binding of the ligand with the G protein coupled receptor protein. With respect to the buffer, any buffer such as Tris-HCl buffer or phosphate buffer of pH 4-10 (preferably, pH 6-8) which does not inhibit the binding of the ligand with the receptor may be used.

In addition, a surface-active agent such as CHAPS, Tween 80™ (Kao-Atlas, Japan), digitonin, deoxycholate, etc. and/or various proteins such as bovine serum albumin (BSA), gelatine, etc. may be added to the buffer with an object of decreasing the nonspecific binding. Further, a protease inhibitor such as PMSF, leupeptin, E-64 (manufactured by Peptide Laboratory, Japan), pepstatin, etc. may be added with an object of inhibiting the decomposition of the receptor and the ligand by protease. A labeled ligand in a certain amount (5,000 cpm to 500,000 cpm) is added to 0.01 ml to 10 ml of said receptor solution and, at the same time, $10^{-4}$ M to $10^{-10}$ M of a test compound coexists. In order to determine the nonspecific binding amount (NSB), a reaction tube to which a great excessive amount of unlabeled test compounds is added is prepared as well.

The reaction is carried out at 0-50°C (preferably at 4-37°C) for 20 minutes to 24 hours (preferably 30 minutes to three hours). After the reaction, it is filtered through a glass fiber filter, a filter paper, or the like, washed with a suitable amount of the same buffer and the radioactivity retained in the glass fiber filter, etc. is measured by means of a liquid scintillation counter or a gamma-counter. Supposing that the count ($B_0$ - NSB) obtained by subtracting the nonspecific binding amount (NSB) from the total binding amount ($B_0$) wherein an antagonizing substance is not present is set at 100%, a test compound in which the specific binding amount (B - NSB) obtained by subtracting the nonspecific binding amount (NSB) from the total binding amount (B) is, for example, less than 50% may be selected as a candidate ligand to the G protein coupled receptor protein of the present invention.

In conducting the above-mentioned methods ④ to ⑤ for screening the compound which inhibits the binding of the ligand with the G protein coupled receptor protein, the G protein coupled receptor protein-mediated cell stimulating activity (e.g. activities of promoting or activities of inhibiting physiological responses such as liberation of arachidonic acid, liberation of acetylcholine, endocellular $Ca^{2+}$ liberation, endocellular cAMP production, production of inositol phosphate, changes in the cell membrane potential, phosphorylation of endocellular proteins, activation of c-fos, lowering of pH, activation of G protein and cell promulgation, etc.) may be measured by known methods or by the use of commercially available measuring kits. To be more specific, G protein coupled receptor protein-containing cells are at first cultured in a multiwell plate or the like.

In conducting the screening, it is substituted with a suitable buffer which does not show toxicity to fresh media or cells in advance, incubated under appropriate conditions and for a specified time after adding a test compound, etc. thereto. The resultant cells are extracted or the supernatant liquid is recovered and the resulting product is determined, preferably quantitatively, by each of the methods. When it is difficult to identify the production of the index substance (e.g. arachidonic acid, etc.) which is to be an index for the cell stimulating activity due to the presence of decomposing enzymes contained in the cell, an assay may be carried out by adding an inhibitor against said decomposing enzyme. With respect to the activities such as an inhibitory action against cAMP production, it may be detected as an inhibitory action against the cAMP production in the cells whose fundamental production has been increased by forskolin or the like.

In conducting a screening by measuring the cell stimulating activity, cells in which a suitable G protein coupled receptor protein is expressed are necessary. Preferred G protein coupled receptor protein-expressing cells are naturally occurring G protein coupled receptor protein (natural type G protein coupled receptor protein)-containing cell lines or strains (e.g. mouse pancreatic β cell line, MIN6, etc.), the above-mentioned recombinant type G protein coupled receptor protein-expressing cell lines or strains, etc.

Examples of the test compound includes peptides, proteins, non-peptidic compounds, synthesized compounds, fermented products, cell extracts, plant extracts, animal tissue extracts, serum, blood, body fluid, etc. Those compounds may be novel or known.

A kit for screening the compound which inhibits the binding of the ligand with the G protein coupled receptor protein or a salt thereof of the present invention comprises a G protein coupled receptor protein or a fragment (partial peptide) thereof, or G protein coupled receptor protein-containing cells or cell membrane fraction thereof.

Examples of the screening kit include as follows:

1. Reagent for Determining Ligand.

① Buffer for Measurement and Buffer for Washing.

The product wherein 0.05% of bovine serum albumin (manufactured by Sigma) is added to Hanks' Balanced Salt Solution (manufactured by Gibco).

This may be sterilized by filtration through a membrane filter with a 0.45 μm pore size, and stored at 4°C or may be prepared upon use.

② Sample of G Protein Coupled Receptor Protein.

CHO cells in which a G protein coupled receptor protein is expressed are subcultured at the rate of $5 \times 10^5$ cells/well in a 12-well plate and cultured at 37°C with a 5% $CO_2$ and 95% air atmosphere for two days to prepare the sample.

③ Labeled Ligand.

The ligand which is labeled with commercially available [$^3$H], [$^{125}$I], [$^{14}$C], [$^{35}$S], etc.

The product in a state of an aqueous solution is stored at 4°C or at -20°C and, upon use, diluted to 1 μM with a buffer for the measurement.

④ Standard Ligand Solution.

Ligand is dissolved in PBS containing 0.1% of bovine serum albumin (manufactured by Sigma) to make 1 mM and stored at -20°C.

2. Method of the Measurement.

① CHO cells are cultured in a 12-well tissue culture plate to express G protein coupled receptor proteins. The G protein coupled receptor protein-expressing CHO cells are washed with 1 ml of buffer for the measurement twice. Then 490 μl of buffer for the measurement is added to each well.
② Five μl of a test compound solution of $10^{-3}$ to $10^{-10}$ M is added, then 5 μl of a labeled ligand is added and is made to react at room temperature for one hour. For knowing the non-specific binding amount, 5 μl of the ligand of $10^{-3}$ M is added instead of the test compound.
③ The reaction solution is removed from the well, which is washed with 1 ml of buffer for the measurement three times. The labeled ligand binding with the cells is dissolved in 0.2N NaOH-1% SDS and mixed with 4 ml of a liquid scintillator A (such as manufactured by Wako Pure Chemical, Japan).
④ Radioactivity is measured using a liquid scintillation counter (e.g., one manufactured by Beckmann) and PMB (percent maximum binding) is calculated by the following equation:

$$PMB = [(B - NSB)/(B_0 - NSB)] \times 100$$

PMB:    Percent maximum binding
B:      Value when a sample is added
NSB:    Nonspecific binding
$B_0$:  Maximum binding

The compound or a salt thereof obtained by the screening method or by the screening kit is a compound which inhibits the binding of a ligand with a G protein coupled receptor protein and, more particularly, it is a compound having a cell stimulating activity mediated via a G protein coupled receptor or a salt thereof (so-called "G protein coupled receptor agonist") or a compound having no said stimulating activity (so-called "G protein coupled receptor antagonist"). Examples of said compound are peptides, proteins, non-peptidic compounds, synthesized compounds, fermented products, etc. and the compound may be novel or known.

Said G protein coupled receptor agonist has the same physiological action as the ligand to the G protein coupled receptor protein has and, therefore, it is useful as a safe and less toxic pharmaceutical composition depending upon said ligand activity.

On the other hand, said G protein coupled receptor antagonist is capable of inhibiting the physiological activity of the ligand to the G protein coupled receptor protein and, therefore, it is useful as a safe and less toxic pharmaceutical composition for inhibiting said ligand activity.

When the compound or the salt thereof obtained by the screening method or by the screening kit is used as the above-mentioned pharmaceutical composition, a conventional means may be applied therefor. The compound or the salt thereof may be orally, parenterally, by inhalation spray, rectally, or topically administered as pharmaceutical compositions or formulations (e.g. powders, granules, tablets, pills, capsules, injections, syrups, emulsions, elixirs, suspensions, solutions, etc.). For example, it may be used by an oral route as tablets (sugar-coated if necessary), capsules, elixirs, microcapsules, etc. or by a parenteral route as injections such as an aseptic solution or a suspension in water or in other pharmaceutically acceptable liquid. The pharmaceutical compositions or formulations may comprise at least one such compound alone or in admixture with pharmaceutically acceptable carriers, adjuvants, vehicles, excipients and/or diluents. The pharmaceutical compositions can be formulated in accordance with conventional methods. For example, said compound or the salt thereof is mixed in a unit dose form which is required for preparing a generally approved pharmaceutical preparations together with a physiologically acceptable carriers, flavoring and/or perfuming agents (fragrances), fillers, vehicles, antiseptics, stabilizers, binders, etc. whereupon the preparation can be manufactured. An amount of the effective component in those preparations is to be in such an extent that the suitable dose within an indicated range is achieved.

Examples of the additives which can be admixed in the tablets, capsules, etc. are binders such as gelatin, corn starch, tragacanth and gum arabicum; fillers such as crystalline cellulose; swelling agents such as corn starch, gelatin and alginic acid; lubricants such as magnesium stearate; sweetening agents such as sucrose, lactose and saccharine; preservatives such as parabens and sorbic acid; antioxidants such as ascorbic acid, $\alpha$-tocopherol and cysteine; fragrances such as peppermint, akamono oil and cherry; disintegrants; buffering agents; etc. Other additives may include mannitol, maltitol, dextran, agar, chitin, chitosan, pectin, collagen, casein, albumin, synthetic or semi-synthetic polymers, glyceride, lactide, etc. When the unit form of the preparation is a capsule, a liquid carrier such as fat/oil may be further added besides the above-mentioned types of materials. The aseptic composition for injection may be formulated by a conventional technique or practice for the preparations such as that the active substance in a vehicle such as water for injection is dissolved or suspended in a naturally occurring plant oil such as sesame oil and palm oil.

Examples of an aqueous liquid for the injection are a physiological saline solution and isotonic solutions containing glucose and other auxiliary agents (e.g. D-sorbitol, D-mannitol, sodium chloride, etc.) wherein a suitable auxiliary solubilizers such as alcohol (e.g. ethanol, etc.), polyalcohol (e.g. propylene glycol, polyethylene glycol, etc.), nonionic surface-active agent (e.g. Polysorbate 80™, HCO-50, etc.), etc. may be jointly used. In the case of the oily liquid, sesame oil, soybean oil, etc. may be exemplified wherein benzyl benzoate, benzyl alcohol, etc. may be jointly used as auxiliary solubilizers.

In addition, buffers (e.g. phosphate buffer, sodium acetate buffer, etc.), analgesic agents (e.g. benzalkonium chloride, procaine hydrochloride, etc.), stabilizers (e.g. human serum albumin, polyethylene glycol, etc.), stabilizers (e.g. benzyl alcohol, phenol, etc.), antioxidants, etc. may be compounded therewith too. The prepared injection solution is filled in suitable ampoules. The formulation prepared as such is safe and less toxic and, therefore, it can be administered to warm-blooded mammals such as rats, rabbits, sheep, swines, cattle, cats, dogs, monkeys, human being, etc.

Dose levels of said compound or the salt thereof may vary depending upon the symptom. Specific dose levels for any particular patient will be employed depending upon a variety of factors including the activity of specific compounds employed, the age, body weight, general health, sex, diet, time of administration, route of administration, rate of excretion, drug combination, and the severity of the particular disease undergoing therapy. In the case of oral administration, it is usually about 0.1-100 mg, preferably about 1.0-50 mg or, more preferably, about 1.0-20 mg per day for adults (as 60 kg). When it is administered parenterally, its dose at a time may vary depending upon the object to be administered, organs to be administered, symptoms, administering methods, etc. The term "parenteral" as used herein includes subcutaneous injections, intravenous, intramuscular, intraperitoneal injections, or infusion techniques. In the case of injections, it is usually convenient to give by an intravenous route in an amount of about 0.01-30 mg, preferably about 0.1-20 mg or, more preferably, about 0.1-10 mg per day to adults (as 60 kg). In the case of other animals, the dose calculated for 60 kg may be administered as well.

(5) Manufacture of Antibody or Antiserum against the G Protein Coupled Receptor Protein of the Present Invention, Its Partial Peptide or Its Salt.

Antibodies (e.g. polyclonal antibody, monoclonal antibody, fragments thereof such as Fab', and single chain antibody) and antisera against the G protein coupled receptor protein or salt thereof of the present invention or against the fragment (partial peptide) of the G protein coupled receptor protein or salt thereof of the present invention may be manufactured by antibody or antiserum-manufacturing methods per se known to those of skill in the art or methods similar thereto, using the G protein coupled receptor protein or its salt of the present invention or the peptide fragment of the G protein coupled receptor protein or its salt of the present invention. For example, monoclonal antibodies can be manufactured by the method as given below.

[Preparation of Monoclonal Antibody]

(a) Preparation of Monoclonal Antibody-Producing Cells.

The G protein coupled receptor protein of the present invention or its salt or the fragment of the G protein coupled receptor protein of the present invention or its salt (hereinafter, may be abbreviated as the "G protein coupled receptor protein") is administered to warm-blooded animals either solely or together with carriers or diluents to a site where the production of antibody is possible by an administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and chickens and the use of mice and rats is preferred.

In the preparation of the cells which produce monoclonal antibodies, an animal wherein the antibody titer is noted is selected from warm-blooded animals (e.g. mice) immunized with antigens, then spleen or lymph node is collected after two to five days from the final immunization and antibody-producing cells contained therein are fused with myeloma cells to give monoclonal antibody-producing hybridomas. Measurement of the antibody titer in antisera may, for example, be carried out by reacting a labeled G protein coupled receptor protein (which will be mentioned later) with the antiserum followed by measuring the binding activity of the labeling agent with the antibody. The operation for fusing may be carried out, for example, by a method of Koehler and Milstein (Nature, 256, 495, 1975). Examples of the fusion accelerator are polyethylene glycol (PEG), Sendai virus, etc. and the use of PEG is preferred.

Examples of the myeloma cells are NS-1, P3U1, SP2/0, AP-1, etc. and the use of P3U1 is preferred. The preferred fusion ratio of the numbers of antibody-producing cells used (spleen cells) to the numbers of myeloma cells is within a range of about 1:1 to 20:1. When PEG (preferably, PEG 1000 to PEG 6000) is added in a concentration of about 10-80% followed by incubating at 20-40°C (preferably, at 30-37°C) for one to ten minutes, an efficient cell fusion can be carried out.

Various methods may be applied for screening a hybridoma which produces anti-G protein coupled receptor antibody. For example, a supernatant liquid of hybridoma culture is added to a solid phase (e.g. microplate) to which the G protein coupled receptor protein antigen is adsorbed either directly or with a carrier, then anti-immunoglobulin antibody (anti-mouse immunoglobulin antibody is used when the cells used for the cell fusion are those of mouse) which is labeled with a radioactive substance, an enzyme or the like, or protein A is added thereto and then anti-G protein coupled receptor monoclonal antibodies bound on the solid phase are detected; or a supernatant liquid of the hybridoma culture is added to the solid phase to which anti-immunoglobulin or protein A is adsorbed, then the G protein coupled receptor labeled with a radioactive substance or an enzyme is added and anti-G protein coupled receptor monoclonal antibodies bonded with the solid phase is detected.

Selection and cloning of the anti-G protein coupled receptor monoclonal antibody-producing hybridoma may be carried out by methods per se known to those of skill in the art or methods similar thereto. Usually, it is carried out in a medium for animal cells, containing HAT (hypoxanthine, aminopterin and thymidine). With respect to a medium for the selection, for the cloning and for the growth, any medium may be used so far as hybridoma is able to grow therein. Examples of the medium are an RPMI 1640 medium (Dainippon Pharmaceutical Co., Ltd., Japan) containing 1-20% (preferably 10-20%) of fetal calf serum (FCS) , a GIT medium (Wako Pure Chemical, Japan) containing 1-20% of fetal calf serum and a serum-free medium for hybridoma culturing (SFM-101; Nissui Seiyaku, Japan). The culturing temperature is usually 20-40°C and, preferably, about 37°C. The culturing time is usually from five days to three weeks and, preferably, one to two weeks. The culturing is usually carried out in 5% carbon dioxide gas. The antibody titer of the supernatant liquid of the hybridoma culture may be measured by the same manner as in the above-mentioned measurement of the antibody titer of the anti-G protein coupled receptor in the antiserum.

The cloning can be usually carried out by methods known per se such as techniques in semi-solid agar and limiting dilution. The cloned hybridoma is preferably cultured in modern serum-free culture media to obtain optimal amounts of antibody in supernatants. The target monoclonal antibody is also preferably obtained from ascitic fluid derived from a mouse, etc. injected intraperitoneally with live hybridoma cells. In another preferred embodiment humanized monoclonal antibodies are used.

(b) Purification of the Monoclonal Antibody.

Like in the separation/purification of conventional polyclonal antibodies, the separation/purification of the anti-G protein coupled receptor monoclonal antibody may be carried out by methods for separating/purifying immunoglobulin (such as salting-out, precipitation with an alcohol, isoelectric precipitation, electrophoresis, adsorption/deadsorption using ion exchangers such as DEAE, ultracentrifugation, gel filtration, specific purifying methods in which only an antibody is collected by treatment with an active adsorbent (such as an antigen-binding solid phase, protein A or protein G) and the bond is dissociated whereupon the antibody is obtained.

The G protein coupled receptor antibody of the present invention which is manufactured by the aforementioned method (a) or (b) is capable of specifically recognizing G protein coupled receptors and, accordingly, it can be used for a quantitative determination of the G protein coupled receptor in test liquid samples and particularly for a quantitative determination by sandwich immunoassays.

Thus, the present invention provides, for example, the following methods:

(i) a quantitative determination of a G protein coupled receptor in a test liquid sample, which comprises

(a) competitively reacting the test liquid sample and a labeled G protein coupled receptor with an antibody which reacts with the G protein coupled receptor of the present invention, and
(b) measuring the ratio of the labeled G protein coupled receptor binding with said antibody; and

(ii) a quantitative determination of a G protein coupled receptor in a test liquid sample, which comprises

(a) reacting the test liquid sample with an antibody immobilized on an insoluble carrier and a labeled antibody simultaneously or continuously, and
(b) measuring the activity of the labeling agent on the insoluble carrier

wherein one antibody is capable of recognizing the N-terminal region of the G protein coupled receptor while another antibody is capable of recognizing the C-terminal region of the G protein coupled receptor.

When the monoclonal antibody of the present invention recognizing a G protein coupled receptor (hereinafter, may be referred to as "anti-G protein coupled receptor antibody") is used, G protein coupled receptors can be measured and, moreover, can be detected by means of a tissue staining, etc. as well. For such an object, antibody molecules per se may be used or F(ab')$_2$, Fab' or Fab fractions of the antibody molecule may be used too. There is no particular limitation for the measuring method using the antibody of the present invention and any measuring method may be used so far as it relates to a method in which the amount of antibody, antigen or antibody-antigen complex, depending on or corresponding to the amount of antigen (e.g. the amount of G protein coupled receptor, etc.) in the liquid sample to be measured, is detected by a chemical or a physical means and then calculated using a standard curve prepared by a standard solution containing the known amount of antigen. For example, nephrometry, competitive method, immunometric method and sandwich method are suitably used and, in terms of sensitivity and specificity, the sandwich method which will be described herein later is particularly preferred.

Examples of the labeling agent used in the measuring method using the labeling substance are radioisotopes, enzymes, fluorescent substances, luminescent substances, colloids, magnetic substances, etc. Examples of the radioisotope are [$^{125}$I], [$^{131}$I], [$^3$H] and [$^{14}$C]; preferred examples of the enzyme are those which are stable and with big specific activity, such as β-galactosidase, β-glucosidase, alkali phosphatase, peroxidase and malate dehydrogenase; examples of the fluorescent substance are fluorescamine, fluorescein isothiocyanate, etc.; and examples of the luminescent substance are luminol, luminol derivatives, luciferin, lucigenin, etc. Further, a biotin-avidin system may also be used for binding an antibody or antigen with a labeling agent.

In an insolubilization (immobilization) of antigens or antibodies, a physical adsorption may be used or a chemical binding which is usually used for insolubilization or immobilization of proteins or enzymes may be used as well. Examples of the carrier are insoluble polysaccharides such as agarose, dextran and cellulose; synthetic resins such as polystyrene, polyacrylamide and silicone; glass; etc.

In a sandwich (or two-site) method, the test liquid is made to react with an insolubilized anti-G protein coupled receptor antibody (the first reaction), then it is made to react with a labeled anti-G protein coupled receptor antibody (the second reaction) and the activity of the labeling agent on the insoluble carrier is measured whereupon the amount of the G protein coupled receptor in the test liquid can be determined. The first reaction and the second reaction may be conducted reversely or simultaneously or they may be conducted with an interval. The type of the labeling agent and the method of insolubilization (immobilization) may be the same as those mentioned already herein. In the immunoassay by means of a sandwich method, it is not always necessary that the antibody used for the labeled antibody and the antibody for the solid phase is one type or one species but, with an object of improving the measuring sensitivity, etc., a mixture of two or more antibodies may be used too.

In the method of measuring G protein coupled receptors by the sandwich method of the present invention, the preferred anti-G protein coupled receptor antibodies used for the first and the second reactions are antibodies wherein their sites binding to the G protein coupled receptors are different each other. Thus, the antibodies used in the first and the second reactions are those wherein, when the antibody used in the second reaction recognizes the C-terminal region of the G protein coupled receptor, then the antibody recognizing the site other than C-terminal regions, e.g. recognizing the N-terminal region, is preferably used in the first reaction.

The anti-G protein coupled receptor antibody of the present invention may be used in a measuring system other than the sandwich method such as a competitive method, an immunometric method and a nephrometry. In a competitive method, an antigen in the test solution and a labeled antigen are made to react with an antibody in a competitive manner, then an unreacted labeled antigen (F) and a labeled antigen binding with an antibody (B) are separated (i.e. B/F separation) and the labeled amount of any of B and F is measured whereupon the amount of the antigen in the test solution is determined. With respect to a method for such a reaction, there are a liquid phase method in which a soluble antibody is used as the antibody and the B/F separation is conducted by polyethylene glycol, a second antibody to the above-mentioned antibody, etc.; and a solid phase method in which an immobilized antibody is used as the first antibody or a soluble antibody is used as the first antibody while an immobilized antibody is used as the second antibody.

In an immunometric method, an antigen in the test solution and an immobilized antigen are subjected to a competitive reaction with a certain amount of a labeled antibody followed by separating into solid and liquid phases; or the antigen in the test solution and an excess amount of labeled antibody are made to react, then a immobilized antigen is added to bind an unreacted labeled antibody with the solid phase and separated into solid and liquid phases. After that, the labeled amount of any of the phases is measured to determine the antigen amount in the test solution.

In a nephrometry, the amount of insoluble sediment which is produced as a result of the antigen-antibody reaction in a gel or in a solution is measured. Even when the antigen amount in the test solution is small and only a small amount of the sediment is obtained, a laser nephrometry wherein scattering of laser is utilized can be suitably used.

In applying each of those immunological measuring methods (immunoassays) to the measuring method of the present invention, it is not necessary to set up any special condition, operation, etc. therefor. A measuring system (assay system) for G protein coupled receptor may be constructed taking the technical consideration of the persons skilled in the art into consideration in the conventional conditions and operations for each of the methods. With details of those conventional technical means, a variety of reviews, reference books, etc. may be referred to. They are, for example, Hiroshi Irie (ed): "Radioimmunoassay" (Kodansha, Japan, 1974); Hiroshi Irie (ed): "Radioimmunoassay, Second Series" (Kodansha, Japan, 1979); Eiji Ishikwa et al. (ed): "Enzyme Immunoassay" (Igaku Shoin, Japan, 1978); Eiji Ishikawa et al. (ed): "Enzyme Immunoassay" (Second Edition) (Igaku Shoin, Japan, 1982); Eiji Ishikawa et al. (ed): "Enzyme Immunoassay" (Third Edition) (Igaku Shoin, Japan, 1987); "Methods in Enzymology" Vol. 70 (Immunochemical Techniques (Part A)); ibid. Vol. 73 (Immunochemical Techniques (Part B)); ibid. Vol. 74 (Immunochemical Techniques (Part C)); ibid. Vol. 84 (Immunochemical Techniques (Part D: Selected Immunoassays)); ibid. Vol. 92 (Immunochemical Techniques (Part E: Monoclonal Antibodies and General Immunoassay Methods)); ibid. Vol. 121 (Immunochemical Techniques (Part I: Hybridoma Technology and Monoclonal Antibodies)) (Academic Press); etc.

As such, the amount of G protein coupled receptor proteins can now be determined with a high precision using the anti-G protein coupled receptor antibody of the present invention.

In the specification and drawings of the present application, the abbreviations used for bases (nucleotides), amino acids and so forth are those recommended by the IUPAC-IUB Commission on Biochemical Nomenclature or those conventionally used in the art. Examples thereof are given below. Amino acids for which optical isomerism is possible are, unless otherwise specified, in the L form.

DNA : Deoxyribonucleic acid
cDNA: Complementary deoxyribonucleic acid
A : Adenine
T : Thymine
G : Guanine
C : Cytosine
RNA : Ribonucleic acid
mRNA : Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP : Adenosine triphosphate
EDTA: Ethylenediamine tetraacetic acid
SDS : Sodium dodecyl sulfate
EIA: Enzyme Immunoassay
G, Gly: Glycine (or Glycyl)
A, Ala: Alanine (or Alanyl)
V, Val: Valine (or Valyl)
L, Leu: Leucine (or Leucyl)

| | |
|---|---|
| I, Ile: | Isoleucine (or Isoleucyl) |
| S, Ser: | Serine (or Seryl) |
| T, Thr: | Threonine (or Threonyl) |
| C, Cys: | Cysteine (or Cysteinyl) |
| M, Met: | Methionine (or Methionyl) |
| E, Glu: | Glutamic acid (or Glutamyl) |
| D, Asp: | Aspartic acid (or Aspartyl) |
| K, Lys: | Lysine (or Lysyl) |
| R, Arg: | Arginine (or Arginyl) |
| H, His: | Histidine (or Histidyl) |
| F, Phe: | Pheylalanine (or Pheylalanyl) |
| Y, Tyr: | Tyrossine (or Tyrosyl) |
| W, Trp: | Tryptophan (or Tryptophanyl) |
| P, Pro: | Proline (or Prolyl) |
| N, Asn: | Asparagine (or Asparaginyl) |
| Q, Gln: | Glutamine (or Glutaminyl) |
| NVal: | Norvaline (or Norvalyl) |
| pGlu: | Pyroglutamic acid (or Pyroglutamyl) |
| Blc: | $\gamma$-Butyrolacton-$\gamma$-carbonyl |
| Kpc: | 2-Ketopiperidinyl-6-carbonyl |
| Otc: | 3-Oxoperhydro-1,4-thiazin-5-carbonyl |
| Me: | Methyl |
| Et: | Ethyl |
| Bu: | Butyl |
| Ph: | Phenyl |
| TC: | Thiazolidinyl-4(R)-carboxamide |

Each SEQ ID NO set forth in the SEQUENCE LISTING of the specification refers to the following sequence:

[SEQ ID NO: 1] is a partial amino acid sequence encoded by the rabbit gastropyrolic part smooth muscle-derived G protein coupled receptor protein cDNA included in pMD4,

[SEQ ID NO: 2] is a full-length amino acid sequence encoded by the rabbit gastropyrolic part smooth muscle-derived G protein coupled receptor protein cDNA included in pUC-C3,

[SEQ ID NO: 3] is a nucleotide sequence of the rabbit gastropyrolic part smooth muscle-derived G protein coupled receptor protein cDNA fragment included in pMD4,

[SEQ ID NO: 4] is a nucleotide sequence of the rabbit gastropyrolic part smooth muscle-derived G protein coupled receptor protein cDNA included in pUC-C3,

[SEQ ID NO: 5] is a synthetic DNA primer for screening of cDNA coding for the G protein coupled receptor protein of the present invention,

[SEQ ID NO: 6] is a synthetic DNA primer for screening of cDNA coding for the G protein coupled receptor protein of the present invention,

[SEQ ID NO: 7] is a synthetic DNA primer for screening of cDNA coding for the G protein coupled receptor protein of the present invention, and

[SEQ ID NO: 8] is a synthetic DNA primer for screening of cDNA coding for the G protein coupled receptor protein of the present invention.

The transformant Escherichia coli, designated JM109/pMD4, which is obtained in the Example 3 mentioned herein below, is on deposit under the terms of the Budapest Treaty from November 11, 1994, 1994, with the National Institute of Bioscience and Human-Technology (NIBH), Agency of Industrial Science and Technology, Ministry of International Trade and Industry, Japan and has been assigned the Accession Number FERM BP-4888. It is also on deposit from November 17, 1994 with the Institute for Fermentation, Osaka, Japan (IFO) and has been assigned the Accession Number IFO 15765.

The transformant Escherichia coli, designated JM109/pUC-C3, which is obtained in the Example 4 mentioned herein below, is on deposit under the terms of the Budapest Treaty from August 10, 1995, with NIBH and has been assigned the Accession Number FERM BP-5198. It is also on deposit from August 4, 1995 with IFO and has been assigned the Accession Number IFO 15858.

The practice of the present invention will employ, unless otherwise indicated, conventional techniques of molecular biology, microbiology, recombinant DNA, pharmacology, immunology, bioscience, and medical technology, which are within the skill of the art. All patents, patent applications, and publications mentioned herein, both supra and infra, are hereby incorporated herein by reference.

EXAMPLES

Described below are working examples of the present invention which are provided only for illustrative purposes, and not to limit the scope of the present invention. In light of the present disclosure, numerous embodiments within the scope of the claims will be apparent to those of ordinary skill in the art. Incidentally, the gene operation using Escherichia coli is carried out by a method described in Maniatis, et al.: "Molecular Cloning" (Cold Spring Harbor Laboratory, 1989).

Reference Example 1

Preparation of Synthetic DNA Primer for Amplifying DNA Coding for G Protein Coupled Receptor Protein

A comparison of deoxyribonucleotide sequences coding for the known amino acid sequences corresponding to or near the first membrane-spanning domain each of human-derived TRH receptor protein (HTRHR), human-derived RANTES receptor protein (L10918, HUMRANTES), human Burkitt's lymphoma-derived unknown ligand receptor protein (X68149, HSBLR1A), human-derived somatostatin receptor protein (L14856, HUMSOMAT), rat-derived $\mu$-opioid receptor protein (U02083, RNU02083), rat-derived $\kappa$-opioid receptor protein (U00442, U00442), human-derived neuromedin B receptor protein (M73482, HUMNMBR), human-derived muscarinic acetylcholine receptor protein (X15266, HSHM4), rat-derived adrenaline $\alpha_1$B receptor protein (L08609, RATAADRE01), human-derived somatostatin 3 receptor protein (M96738, HUMSSTR3X), human-derived $C_5$ a receptor protein (HUMC5AAR), human-derived unknown ligand receptor protein (HUMRDC1A), human-derived unknown ligand receptor protein (M84605, HUMOPIODRE) and rat-derived adrenaline $\alpha_2$B receptor protein (M91466, RATA2BAR) was made. As a result, highly homologous regions or parts were found.

Further, a comparison of deoxynucleotide sequences coding for the known amino acid sequences corresponding to or near the sixth membrane-spanning domain each of mouse-derived unknown ligand receptor protein (M80481, MUSGIR), human-derived bombesin receptor protein (L08893, HUMBOMB3S), human-derived adenosine A2 receptor protein (S46950, S46950), mouse-derived unknown ligand receptor protein (D21061, MUSGPCR), mouse-derived TRH receptor protein (S43387, S43387), rat-derived neuromedin K receptor protein (J05189, RATNEURA), rat-derived adenosine A1 receptor protein (M69045, RATA1ARA), human-derived neurokinin A receptor protein (M57414, HUMNEKAR), rat-derived adenosine A3 receptor protein (M94152, RATADENREC), human-derived somatostatin 1 receptor protein (M81829, HUMSRI1A), human-derived neurokinin 3 receptor protein (S86390, S86371S4), rat-derived unknown ligand receptor protein (X61496, RNCGPCR), human-derived somatostatin 4 receptor protein (L07061, HUMSSTR4Z) and rat-derived GnRH receptor protein (M31670, RATGNRHA) was made. As a result, highly homologous regions or parts were found.

The aforementioned abbreviations in the parentheses are identifiers (reference numbers) which are indicated when GenBank/EMBL Data Bank is retrieved by using DNASIS Gene/Protein Sequencing Data Base (CD019, Hitachi Software Engineering, Japan) and are usually called "Accession Numbers" or "Entry Names". HTRHR is, however, the sequence as disclosed in Japanese Unexamined Patent Publication No. 286986/1993 (EPA 638645).

Specifically, it was planned to incorporate mixed bases relying upon the base regions that were in agreement with cDNAs coding for a large number of receptor proteins in order to enhance base agreement of sequences with as many receptor cDNAs as possible even in other regions. Based upon these sequences, the degenerate synthetic DNA having a nucleotide sequence represented by SEQ ID NO: 5 which is complementary to the homologous nucleotide sequence and the degenerate synthetic DNA having a nucleotide sequence represented by SEQ ID NO: 6 which is complementary to the homologous nucleotide sequence were produced. Nucleotide synthesis was carried out by a DNA synthesizer.

[Synthetic DNAs]

5'-CGTGG (G or C) C (A or C) T (G or C) (G or C) TGGGCAAC
    (A, G, C or T) (C or T) CCTG-3'

(SEQ ID NO: 5)

5'-GT (A, G, C or T) G (A or T) (A or G) (A or G) GGCA
    (A, G, C or T) CCAGCAGA (G or T) GGCAAA-3'

(SEQ ID NO: 6)

The parentheses indicate the incorporation of a plurality of bases, leading to multiple oligonucleotides in the primer preparation. In other words, nucleotide residues in parentheses of the aforementioned DNAs were incorporated in the presence of a mixture of plural bases at the time of synthesis.

Example 1

Preparation of Poly(A)⁺RNA Fraction from Rabbit Gastropyrolic Part Smooth Muscle and Synthesis of cDNA

A total RNA was prepared from rabbit gastropyrolic part smooth muscles by the guanidine thiocyanate method (Kaplan B.B. et al., Biochem. J. 183, 181-184 (1979)) and, then, poly(A)⁺RNA fractions were prepared with a mRNA purifying kit (Pharmacia Co.). Next, to 5 µg of the poly(A)⁺RNA fraction was added a random DNA hexamer (BRL Co.) as a primer, and the resulting mixture was subjected to reaction with mouse Moloney Leukemia virus (MMLV) reverse transcriptase (BRL Co.) in the buffer attached to the MMLV reverse transcriptase kit to synthesize complementary DNAs. The reaction product was extracted with phenol/chloroform (1:1), precipitated in ethanol, and was then dissolved in 30 µl of TE buffer (Tris-EDTA solution; 10 mM Tris-HCl at pH8.0, 1 mM EDTA at pH8.0).

Example 2

Amplification of Receptor cDNA by PCR Using Rabbit Gastropyrolic Part Smooth Muscle-Derived cDNA and Sequencing

By using, as a template, 1 µl of cDNA prepared from the rabbit gastropyrolic part smooth muscle in Example 1, PCR amplification using the DNA primers synthesized in Reference Example 1 was carried out. A reaction solution was composed of the synthetic DNA primers (SEQ: 5' primer sequence and 3' primer sequence) each in an amount of 1 µM, 0.25 mM dNTPs (deoxyribonucleotide triphosphates), 1 µl of Taq DNA polymerase (Takara Shuzo Co., Japan) and 10 µl of buffer attached to the enzyme kit, and the total amount of the reaction solution was made to be 100 µl. The cycle for amplification including 96 °C for 30 sec., 45 °C for 1 min. and 60 °C for 3 min. was repeated 25 times by using a Thermal Cycler (Perkin-Elmer Co.). The amplified products were confirmed relying upon 1.2% agarose gel electrophoresis and ethidium bromide staining.

Example 3

Subcloning of PCR Product into Plasmid Vector and Selection of Novel Receptor Candidate Clone via Decoding Nucleotide Sequence of Inserted cDNA Region

The PCR products obtained in Example 2 were separated with a 1.0% low-melting temperature agarose gel, the band parts were excised from the gel with a razor blade, and were heat-melted, extracted with phenol and precipitated in ethanol to recover DNAs. According to the protocol attached to a TA Cloning Kit (Invitrogen Co.), the recovered DNAs were subcloned to the plasmid vector, pCR™II. The recombinant vectors were introduced into E. coli JM109 competent cells (Takara Shuzo Co., Japan) to produce transformants. Then, transformant clones having a cDNA-inserted fragment were selected in an LB (Luria-Bertani) agar culture medium containing ampicillin, IPTG (isopropylthio-β-D-galactoside) and X-gal (5-bromo-4-chloro-3-indolyl-β-D-galactoside). Only transformant clones exhibiting white color were picked with sterilized toothsticks to obtain transformant Escherichia coli JM109/pMD4.

The individual clones were cultured overnight in an LB culture medium containing ampicillin and treated with an automatic plasmid extracting machine (Kurabo Co., Japan) to prepare plasmid DNAs. An aliquot of the DNAs thus prepared was cut by EcoRI to confirm the size of the cDNA insert. An aliquot of the remaining DNAs was further processed with RNase, extracted with phenol/chloroform, and precipitated in ethanol so as to be condensed. Sequencing was carried out by using a DyeDeoxy terminator cycle sequencing kit (ABI Co.), the DNAs were decoded by using a fluorescent automatic sequencer, and the data of the nucleotide sequences obtained were read by using DNASIS (Hitachi System Engineering Co., Japan). The determined nucleotide sequence was as shown in FIG. 1 (SEQ ID NO: 1). It was learned from FIG. 1 that the cloned cDNA fragment was amplified from both sides with only the synthetic DNA primer having a nucleotide sequence represented by SEQ ID NO: 5 as synthesized in Reference Example 1.

Database was searched based upon the determined nucleotide sequence [FIG. 1]. As a result, it was learned that a novel G protein coupled receptor protein was encoded by the cDNA insert in the plasmid possessed by the transformant Escherichia coli JM109/pMD4. To further confirm this fact, by using DNASIS (Hitachi System Engineering Co., Japan) the nucleotide sequence were converted into an amino acid sequence [FIG. 1] (SEQ ID NO: 1), and homology retrieval was carried out in view of hydrophobicity plotting [FIG. 2] and at the amino acid sequence level to find similarity to rat ligand-unknown receptor protein (A35639) [FIG. 3]. Abbreviations in parentheses are reference numbers assigned when they are registered as data to NBRF-PIR/Swiss-PROT and are usually called "Accession Numbers".

Example 4

Cloning of cDNA Comprising Whole Coding Regions for Receptor Protein from Rabbit Gastropyrolic Part Smooth Muscle-Derived cDNA Library

To 10 μg of rabbit gastropyrolic part smooth muscle-derived poly(A)⁺RNA prepared in Example 1 was added a random 9mer primer, and the resulting mixture was subjected to reaction according to the manual of a cDNA Synthesis System Plus (Pharmacia) to produce a first strand cDNA, followed by synthesis of a second strand cDNA (double stranded cDNA; 269 ng). By a cDNA rapid adaptor ligation module (Amersham), adaptors were ligated to both ends of the resultant double stranded cDNA according to the manual. Next, by a cDNA rapid cloning module (Amersham), λgt11 vector arms were ligated to 75 ng of the double stranded cDNA according to the manual. Among the cDNA library products, a cDNA library with 2.1 x 10⁶ pfu (plaque forming units) was mixed with E. coli Y1090⁻ treated with magnesium sulfate, and incubated at 37 °C for 15 minutes, followed by addition of 0.5% agarose (Pharmacia Co.) LB. The E. coli was plated onto a 1.5% agar (Wako Pure chemical Co.) LB plate (containing 50 μg/ml of ampicillin). A nitrocellulose filter was placed on the plate on which plaques were formed and the plaques were transferred onto the filter. The filters were denatured with an alkali and then baked at 80 °C for 3 hours to fix DNAs.

The filters were incubated overnight at 42 °C together with the probe mentioned herein below in a buffer containing 50% formamide, 5 x SSPE (SSPE; 150 mM NaCl, 10 mM NaH₂PO₄ · H₂O, 1.25 mM EDTA (pH 7.4)), 5 X Denhardt's solution (Nippon Gene, Japan), 0.1% SDS (sodium dodecyl sulfate) and 100 μg/ml of salmon sperm DNA for hybridization.

The probe used was obtained by cutting the DNA fragment inserted in the plasmid, pMD4, obtained in Example 3, with EcoRI, followed by recovery and labeling by incorporation of [³²P]dCTP (Dupont/NEN) with a random prime DNA labelling kit (Amersham Co.). It was washed with 2 x SSC (SSC; 150 mM NaCl and 15 mM sodium citrate), 0.1% SDS at room temperature for 1.25 hour and, then, 60 °C for 1.25 hour, and subjected to an autoradiography at -80 °C to detect hybridized plaques.

In this screening, hybridization signals were recognized in 98 independent plaques. These clones were picked up and stirred well with 5 ml of SM (50 mM Tris-HCl at pH7.5, 0.1 M NaCl, 7 mM MgSO₄ and 0.01% gelatin). An aliquot of the extract was mixed with E. coli Y1090⁻ treated with magnesium sulfate, and incubated at 37 °C for 15 minutes, followed by addition of 0.5% agarose (Pharmacia Co.) LB. The E. coli was plated onto a 1.5% agar (Wako Pure chemical Co.) LB plate (containing 50 μg/ml of ampicillin). A nitrocellulose filter was placed on the plate on which plaques were formed and the plaques were transferred onto the filter. The filters were denatured with an alkali in the same manner as mentioned above and then baked at 80 °C for 3 hours to fix DNAs.

The filters were hybridized with the same probe in the same manner as mentioned above and washed in the same manner to detect hybridized plaques. In this screening, it was recognized that hybridization signals were positive in 62 independent clones. All these positive clones were picked up for plaques and suspended in water, and centrifuged to obtain a supernatant. The supernatant was heated at 95 °C for 5 min., then rapidly cooled and centrifuged to obtain a supernatant.

By using, as a template, the resultant supernatant, PCR amplification using λ forward primers and λ reverse primers (Takara, Japan) which are based on the sequence in λgt11 phage vector was carried out for determination of its insert size. The cycle for PCR amplification including 95 °C for 45 sec., 52 °C for 1 min. and 72 °C for 3 min. was repeated 25 times, and incubated for 10 min. at 72 °C following the last cycle. As a result, it was considered that 14 clones possess an insert with 1 kbp or more.

Two oligonucleotides (MD4F and MD4R) were synthesized based on a nucleotide sequence of pMD4:

MD4F:

5'-TCGGC TTCTC CATCA AGAGG ACCC-3'          (SEQ ID NO: 7)

MD4R:

5'-CACGC TGCGC ACATA GTGGG CGAA-3'          (SEQ ID NO: 8)

PCR amplification using the two synthetic primers, MD4F and MD4R, together with the λ forward primer and the λ reverse primer was carried out. The cycle for PCR amplification including 95 °C for 45 sec., 58 °C for 1 min. and 72 °C for 1 min. was repeated 30 times, and incubated for 10 min. at 72 °C following the last cycle.

As a result, it was deduced that a clone, named "C-3", possesses a full-length coding region. The inserted cDNA fragment was excised by EcoRI from the clone C-3. About 1.4 kbp cDNA fragment was subcloned into the EcoRI site of pUC18 and the resulting plasmid was transfected into <u>Escherichia</u> <u>coli</u> JM109 to produce a transformant <u>E.</u> <u>coli</u> JM109/pUC-C3. A rabbit gastropyrolic part smooth muscle-derived DNA fragment with about 1.4 kbp in the plasmid pUC-C3 was sequenced.

In brief, by utilizing restriction enzyme sites that exist in the EcoRI fragment, unnecessary parts were removed or necessary fragments were subcloned in order to prepare template plasmids for analyzing the nucleotide sequence. Sequencing was conducted by a Dye Deoxy Terminator Cycle Sequencing kit (ABI Co.), the DNAs were decoded by a fluorescent automatic DNA sequencer (ABI Co.), and the data of the nucleotide sequence obtained were analyzed by a DNASIS (Hitachi System Engineering Co., Japan).

The determined nucleotide sequence of the rabbit gastropyrolic part smooth muscle-derived cDNA fragment which is inserted into pUC-C3 is as shown in FIG. 4. It was learned that the nucleotide sequence of the rabbit gastropyrolic part smooth muscle-derived receptor protein-encoding DNA (SEQ ID NO: 4) corresponds to the nucleotide sequence of from 202nd to 1230th nucleotides in FIG. 4. An amino acid sequence (SEQ ID NO: 2) encoded the cDNA fragment is as shown in FIG. 4. It is recognized that this amino acid sequence has 83% identity relative to rat ligand-unknown receptor protein disclosed in Proc. Natl. Acad. Sci. USA, Vol. 87, pp. 3052-3056, 1990.

Hydrophobicity plotting was conducted at the amino acid sequence level to give FIG. 5.

The G protein coupled receptor protein of the present invention and the DNA coding for said protein can be used for ① assays for the screening and determination of ligands; ② acquisition of antibody and antiserum; ③ construction of expression system for of a recombinant type receptor protein; ④ development of the receptor binding assay system using said expression system and screening of the candidate compounds for pharmaceuticals; ⑤ conducting a drug design based upon a comparison with structurally analogous ligands and receptors; ⑥ preparation of probes and PCR primers for a gene diagnosis; ⑦ preparation of transgenic animals; and ⑧ preparation of model patient animals deficient in the receptor protein DNA. Elucidation of the structure and property of the G protein coupled receptor is particularly related to the development of unique pharmaceuticals which act on such a system.

**Claims**

1. A G protein coupled receptor protein comprising an amino acid sequence selected from the group consisting of an amino acid sequence represented by SEQ ID NO: 1 or SEQ ID NO: 2 and its substantial equivalents thereto, or a salt thereof.

2. A DNA which comprises a nucleotide sequence coding for the G protein coupled receptor protein of Claim 1.

3. The DNA according to Claim 2 comprising a nucleotide sequence represented by SEQ ID NO: 3 or SEQ ID NO: 4.

4. A vector comprising the DNA of Claim 2.

5. A transformant carrying the vector of Claim 4.

6. A process for producing a G protein coupled receptor protein or a salt thereof according to Claim 1, which comprises culturing the transformant of Claim 5 under sufficient conditions and for appropriate time to express said G protein coupled receptor protein.

7. The process of Claim 6, which further comprises allowing said G protein coupled receptor protein or a salt thereof to accumulate, and collecting said G protein coupled receptor protein or a salt thereof.

8. A method of screening for a ligand to the G protein coupled receptor protein according to Claim 1, which comprises contacting (i) the G protein coupled receptor protein or a salt thereof of Claim 1, with (ii) a sample to be tested.

9. A screening method for a compound capable of inhibiting the binding of the G protein coupled receptor protein of Claim 1 with a ligand, which comprises conducting a comparison between:

(i) at least one case where said ligand is contacted with the G protein coupled receptor protein or a salt thereof according to Claim 1, and
(ii) at least one case where said ligand together with a sample to be tested is contacted with said G protein coupled receptor protein or a salt thereof according to Claim 1 and determining the difference in binding activity.

10. A kit for the screening of a compound capable of inhibiting the binding of said G protein coupled receptor protein according to Claim 1 with a ligand, which comprises the G protein coupled receptor protein or a salt thereof according to Claim 1.

11. An antibody which specifically binds to said G protein coupled receptor protein or a salt thereof according to Claim 1.

12. A reagent for probing a G protein coupled receptor protein, which comprises said DNA according to Claim 2.

# FIG. 1

EP 0 711 831 A2

```
                  10            19            28            37            46            55
    5'  GTG GGC ATG GTG GGC AAC GTC CTG GTG CTC TGG TTC TTC GGC TTC TCC ATC AAG
        --- --- --- --- --- --- --- --- --- --- --- --- --- --- --- --- --- ---
        Val Gly Met Val Gly Asn Val Leu Val Leu Trp Phe Phe Gly Phe Ser Ile Lys

                  64            73            82            91            100           109
        AGG ACC CCC TTC TCC GTC TAC TTC CTG CAC CTG GCC AGC GCC GAC GGC GCC TAC
        --- --- --- --- --- --- --- --- --- --- --- --- --- --- --- --- --- ---
        Arg Thr Pro Phe Ser Val Tyr Phe Leu His Leu Ala Ser Ala Asp Gly Ala Tyr

                  118           127           136           145           154           163
        CTC TTC AGC AAG GCC GTG TTC TCC CTG CTG AAC GCC GGC GGC TTC CTG GGC ACC
        --- --- --- --- --- --- --- --- --- --- --- --- --- --- --- --- --- ---
        Leu Phe Ser Lys Ala Val Phe Ser Leu Leu Asn Ala Gly Gly Phe Leu Gly Thr

                  172           181           190           199           208           217
        TTC GCC CAC TAT GTG CGC AGC GTG GCC CGG GTG CTG GGG CTC TGC GCC TTC GTG
        --- --- --- --- --- --- --- --- --- --- --- --- --- --- --- --- --- ---
        Phe Ala His Tyr Val Arg Ser Val Ala Arg Val Leu Gly Leu Cys Ala Phe Val

                  226           235           244           253           262
        GCG GGC GTG AGC CTC CTG CCG GCC GTG AGC ATG GAG CGC TGC GCG TCT G 3'
        --- --- --- --- --- --- --- --- --- --- --- --- --- --- --- --- -
        Ala Gly Val Ser Leu Leu Pro Ala Val Ser Met Glu Arg Cys Ala Ser
```

36

FIG. 2

EP 0 711 831 A2

# FIG. 3

```
                10        20        30        40        50
pMD4    1 VGMVGNVLVL WFFGFSIKRT PFSVYFLHLA SADGAYLFSK AVFSLINAGG   50
A35639  1 CGLVGNGLVL WFFGFSIKRT PFSIYFLHLA SADGIYLFSK AVIALLNMGT   50

                60        70        80        90        100
pMD4   51 FLGTFAHYVR SVARVLGLCA FVAGVSLLPA VSMERCAS.. ..........  100
A35639 51 FLGSFPDYVR RVSRIVGLCT FFAGVSLLPA ISIERCVS.. ..........  100
```

# FIG. 4

```
   1 AGCAAGTGTCCAACTACTTGAGCCATCAGCAGCTGTCTTTTCAGGTACAGTAACAGAAGG          60

  61 ATGTATTGAAAGTGGAAAAACCAGGCCGCCTTTTTCAGATCGTCCCTGGCAGGACAAGAG         120

 121 CCAGGCCACACAAGAAGAGGGAAACCAGCCGCAGAGGCAGGTGCAGGCCGGGAGCTGTGA         180

 181 GCGGTGAGCAGGCACCTGGACATGGCTGAGAACTGCTCCTGGGAGGCGCATCCCACCAAC         240
                             MetAlaGluAsnCysSerTrpGluAlaHisProThrAsn      13

 241 AGGAACAAGGTGTGTCCCGGCGTGAGCGAGGCCCCGGAGCTCTACAGCCGGGGCTTCCTG         300
     ArgAsnLysValCysProGlyValSerGluAlaProGluLeuTyrSerArgGlyPheLeu      33

 301 ACCATCGAGCCGATCGCGCCGCTGCCGCCACCGGCGGTCATGGACTACATCTTCCTGCTC         360
     ThrIleGluProIleAlaProLeuProProProAlaValMetAspTyrIlePheLeuLeu      53

 361 CTCTGCCTGTGCGGCCTGGTGGGCAACGGCCTGGTGCTCTGGTTCTTCGGCTTCTCCATC         420
     LeuCysLeuCysGlyLeuValGlyAsnGlyLeuValLeuTrpPhePheGlyPheSerIle      73

 421 AAGAGGACCCCCTTCTCCGTCTACTTCCTGCACCTGGCCAGCGCCGACGGCGCCTACCTC         480
     LysArgThrProPheSerValTyrPheLeuHisLeuAlaSerAlaAspGlyAlaTyrLeu      93

 481 TTCAGCAAGGCCGTGTTCTCCCTGCTGAACGCCGGCGGCTTCCTGGGCACCTTCGCCCAC         540
     PheSerLysAlaValPheSerLeuLeuAsnAlaGlyGlyPheLeuGlyThrPheAlaHis     113

 541 TATGTGCGCAGCGTGGCCCGGGTGCTGGGGCTCTGCGCCTTCGTGGCGGGCGTGAGCCTC         600
     TyrValArgSerValAlaArgValLeuGlyLeuCysAlaPheValAlaGlyValSerLeu     133

 601 CTGCCGGCCGTGAGCATGGAGCGCTGCGCGTCTGTCGTCTTCCCCGCCTGGTACTGGCGC         660
     LeuProAlaValSerMetGluArgCysAlaSerValValPheProAlaTrpTyrTrpArg     153

 661 CGGCGGCCCAGGCGCCTGTCGGCTGTGGCGTGCGCCCTGCTCTGGCTGCTGGCACTGCTG         720
     ArgArgProArgArgLeuSerAlaValAlaCysAlaLeuLeuTrpLeuLeuAlaLeuLeu     173

 721 GTCACCGGCGTCCACAACTACTTCTGCGTCTTCCTGGGCCGCGAGGCCTCCGGGGGCGGC         780
     ValThrGlyValHisAsnTyrPheCysValPheLeuGlyArgGluAlaSerGlyGlyGly     193

 781 TGCAGGCACACCGACGTCTTCCTGGGCATCTTGCTCTTCCTCGTCTTCTGCCCGCTCATG         840
     CysArgHisThrAspValPheLeuGlyIleLeuLeuPheLeuValPheCysProLeuMet     213

 841 GTGCTGCCCTGCCTGGCCCTCGTGCTGCACGTGGAGTGCCGGGCGCGGCGGCGCCAGCGC         900
     ValLeuProCysLeuAlaLeuValLeuHisValGluCysArgAlaArgArgArgGlnArg     233

 901 TCGGCCAAGCTCAACCACGTGGTCCTGGCCATGGTCTCCGTCTTCCTCGTGTCCTCCATC         960
     SerAlaLysLeuAsnHisValValLeuAlaMetValSerValPheLeuValSerSerIle     253

 961 TACCTGGGCATCGACTGGTTCCTCTTCTGGGTCTTCCAGATCCCCGCGCCCTTCCCCGAG        1020
     TyrLeuGlyIleAspTrpPheLeuPheTrpValPheGlnIleProAlaProPheProGlu     273

1021 TACGTCACGGACCTGTGCATCTGCATCCACAGTGGCGCCAAGCCCGTGGTGTACTTCCTG        1080
     TyrValThrAspLeuCysIleCysIleHisSerGlyAlaLysProValValTyrPheLeu     293

1081 GCCGGCAGGGACAAGTCGCAGCGCCTCTGGGAGCCCCTTAGGGTGGTCTTCCAGCGGGCC        1140
     AlaGlyArgAspLysSerGlnArgLeuTrpGluProLeuArgValValPheGlnArgAla     313

1141 CTGCGCGACGGCGCCGAGCCGGCCGAGCCCGCGGCCAGCACCCCCAACACGGTCACCATG        1200
     LeuArgAspGlyAlaGluProAlaGluProAlaAlaSerThrProAsnThrValThrMet     333

1201 GAGATGCAGGGCCCCTCCGGGAACGCCTCGTGAGGGGCCTGGCGGGCCAGGGCTGCCTCG        1260
     GluMetGlnGlyProSerGlyAsnAlaSer***                               344

1261 GACGGGTGCTCTAGCCCCCAGGCCTGCTTCTCCCAGGGCGGCACGCTCCAAGGGTGGCCG        1320

1321 AGAGACTGAGCAGCCACCTGCAAACGGACGCCGTGGCCTGTCTCCTCCTTAGGGACCCTT        1380

1381 TGCACCAAGCCCGGCCCCGCC                                            1401
```

# FIG. 5

SEQUENCE LISTING

(1) GENERAL INFORMATION:

    (i) APPLICANT:
        (A) NAME:      Takeda Chemical Industries, Ltd.
        (B) STREET:    1-1, Doshomachi 4-chome, Chuo-ku
        (C) CITY:      Osaka-shi
        (D) STATE:     Osaka
        (E) COUNTRY:   Japan
        (F) POSTAL CODE (ZIP):       541

    (ii) TITLE OF INVENTION: G protein coupled receptor protein,
                             production and use thereof

    (iii) NUMBER OF SEQUENCES:              8

(2) INFORMATION FOR SEQ ID NO: 1:

    (i)   SEQUENCE CHARACTERISTICS:
        (A) LENGTH:       71
        (B) TYPE:         Amino acid
        (C) TOPOLOGY:     Linear

    (ii) MOLECULE TYPE:     Peptide

    (xi) SEQUENCE DESCRIPTION:   SEQ ID NO: 1:

Val Leu Trp Phe Phe Gly Phe Ser Ile Lys Arg Thr Pro Phe Ser Val
1               5                   10                  15

Tyr Phe Leu His Leu Ala Ser Ala Asp Gly Ala Tyr Leu Phe Ser Lys
            20                  25                  30

Ala Val Phe Ser Leu Leu Asn Ala Gly Gly Phe Leu Gly Thr Phe Ala
            35                  40                  45

His Tyr Val Arg Ser Val Ala Arg Val Leu Gly Leu Cys Ala Phe Val
        50                  55                  60

Ala Gly Val Ser Leu Leu Pro
65                  70

(2) INFORMATION FOR SEQ ID NO: 2:

    (i)   SEQUENCE CHARACTERISTICS:
        (A) LENGTH:       343
        (B) TYPE:         Amino acid
        (C) TOPOLOGY:     Linear

    (ii) MOLECULE TYPE:     Peptide

    (xi) SEQUENCE DESCRIPTION:   SEQ ID NO: 2:

Met Ala Glu Asn Cys Ser Trp Glu Ala His Pro Thr Asn Arg Asn Lys
1               5                   10                  15

30

```
Val Cys Pro Gly Val Ser Glu Ala Pro Glu Leu Tyr Ser Arg Gly Phe
            20                  25                  30

Leu Thr Ile Glu Pro Ile Ala Pro Leu Pro Pro Pro Ala Val Met Asp
            35                  40                  45

Tyr Ile Phe Leu Leu Leu Cys Leu Cys Gly Leu Val Gly Asn Gly Leu
        50                  55                  60

Val Leu Trp Phe Phe Gly Phe Ser Ile Lys Arg Thr Pro Phe Ser Val
65                  70                  75                  80

Tyr Phe Leu His Leu Ala Ser Ala Asp Gly Ala Tyr Leu Phe Ser Lys
                85                  90                  95

Ala Val Phe Ser Leu Leu Asn Ala Gly Gly Phe Leu Gly Thr Phe Ala
            100                 105                 110

His Tyr Val Arg Ser Val Ala Arg Val Leu Gly Leu Cys Ala Phe Val
        115                 120                 125

Ala Gly Val Ser Leu Leu Pro Ala Val Ser Met Glu Arg Cys Ala Ser
    130                 135                 140

Val Val Phe Pro Ala Trp Tyr Trp Arg Arg Arg Pro Arg Arg Leu Ser
145                 150                 155                 160

Ala Val Ala Cys Ala Leu Leu Trp Leu Leu Ala Leu Leu Val Thr Gly
                165                 170                 175

Val His Asn Tyr Phe Cys Val Phe Leu Gly Arg Glu Ala Ser Gly Gly
            180                 185                 190

Gly Cys Arg His Thr Asp Val Phe Leu Gly Ile Leu Leu Phe Leu Val
        195                 200                 205

Phe Cys Pro Leu Met Val Leu Pro Cys Leu Ala Leu Val Leu His Val
    210                 215                 220

Glu Cys Arg Ala Arg Arg Arg Gln Arg Ser Ala Lys Leu Asn His Val
225                 230                 235                 240

Val Leu Ala Met Val Ser Val Phe Leu Val Ser Ser Ile Tyr Leu Gly
                245                 250                 255

Ile Asp Trp Phe Leu Phe Trp Val Phe Gln Ile Pro Ala Pro Phe Pro
            260                 265                 270

Glu Tyr Val Thr Asp Leu Cys Ile Cys Ile His Ser Gly Ala Lys Pro
        275                 280                 285

Val Val Tyr Phe Leu Ala Gly Arg Asp Lys Ser Gln Arg Leu Trp Glu
    290                 295                 300

Pro Leu Arg Val Val Phe Gln Arg Ala Leu Arg Asp Gly Ala Glu Pro
```

31

```
          305                 310                315                320

Ala Glu Pro Ala Ala Ser Thr Pro Asn Thr Val Thr Met Glu Met Gln
                      325                 330                335

Gly Pro Ser Gly Asn Ala Ser
                340
```

(2) INFORMATION FOR SEQ ID NO: 3:

    (i)   SEQUENCE CHARACTERISTICS:
        (A) LENGTH:      215
        (B) TYPE:        Nucleic acid
        (C) STRANDEDNESS: Double
        (D) TOPOLOGY:    Linear

    (ii) MOLECULE TYPE:   cDNA

    (ix) FEATURE
        (C) IDENTIFICATION METHOD:  S

    (xi) SEQUENCE DESCRIPTION:  SEQ ID NO: 3:

```
GTGCTCTGGT TCTTCGGCTT CTCCATCAAG AGGACCCCCT TCTCCGTCTA CTTCCTGCAC    60

CTGGCCAGCG CCGACGGCGC CTACCTCTTC AGCAAGGCCG TGTTCTCCCT GCTGAACGCC   120

GGCGGCTTCC TGGGCACCTT CGCCCACTAT GTGCGCAGCG TGGCCCGGGT GCTGGGGCTC   180

TGCGCCTTCG TGGCGGGCGT GAGCCTCCTG CCGGC                              215
```

(2) INFORMATION FOR SEQ ID NO: 4:

    (i)   SEQUENCE CHARACTERISTICS:
        (A) LENGTH:      1029
        (B) TYPE:        Nucleic acid
        (C) STRANDEDNESS: Double
        (D) TOPOLOGY:    Linear

    (ii) MOLECULE TYPE:   cDNA

    (ix) FEATURE
        (C) IDENTIFICATION METHOD:  S

    (xi) SEQUENCE DESCRIPTION:  SEQ ID NO: 4:

```
ATGGCTGAGA ACTGCTCCTG GGAGGCGCAT CCCACCAACA GGAACAAGGT GTGTCCCGGC    60

GTGAGCGAGG CCCCGGAGCT CTACAGCCGG GGCTTCCTGA CCATCGAGCC GATCGCGCCG   120

CTGCCGCCAC CGGCGGTCAT GGACTACATC TTCCTGCTCC TCTGCCTGTG CGGCCTGGTG   180

GGCAACGGCC TGGTGCTCTG GTTCTTCGGC TTCTCCATCA AGAGGACCCC CTTCTCCGTC   240

TACTTCCTGC ACCTGGCCAG CGCCGACGGC GCCTACCTCT TCAGCAAGGC CGTGTTCTCC   300
```

```
CTGCTGAACG CCGGCGGCTT CCTGGGCACC TTCGCCCACT ATGTGCGCAG CGTGGCCCGG    360

GTGCTGGGGC TCTGCGCCTT CGTGGCGGGC GTGAGCCTCC TGCCGGCCGT GAGCATGGAG    420

CGCTGCGCGT CTGTCGTCTT CCCCGCCTGG TACTGGCGCC GGCGGCCCAG GCGCCTGTCG    480

GCTGTGGCGT GCGCCCTGCT CTGGCTGCTG GCACTGCTGG TCACCGGCGT CCACAACTAC    540

TTCTGCGTCT TCCTGGGCCG CGAGGCCTCC GGGGGCGGCT GCAGGCACAC CGACGTCTTC    600

CTGGGCATCT TGCTCTTCCT CGTCTTCTGC CCGCTCATGG TGCTGCCCTG CCTGGCCCTC    660

GTGCTGCACG TGGAGTGCCG GGCGCGGCGG CGCCAGCGCT CGGCCAAGCT CAACCACGTG    720

GTCCTGGCCA TGGTCTCCGT CTTCCTCGTG TCCTCCATCT ACCTGGGCAT CGACTGGTTC    780

CTCTTCTGGG TCTTCCAGAT CCCCGCGCCC TTCCCCGAGT ACGTCACGGA CCTGTGCATC    840

TGCATCCACA GTGGCGCCAA GCCCGTGGTG TACTTCCTGG CCGGCAGGGA CAAGTCGCAG    900

CGCCTCTGGG AGCCCCTTAG GGTGGTCTTC CAGCGGGCCC TGCGCGACGG CGCCGAGCCG    960

GCCGAGCCCG CGGCCAGCAC CCCCAACACG GTCACCATGG AGATGCAGGG CCCCTCCGGG    1020

AACGCCTCG                                                           1029
```

(2) INFORMATION FOR SEQ ID NO: 5:

    (i)   SEQUENCE CHARACTERISTICS:
        (A) LENGTH:       25
        (B) TYPE:        Nucleic acid
        (C) STRANDEDNESS: Single
        (D) TOPOLOGY:    Linear

    (ii) MOLECULE TYPE:    Other nucleic acid
                       Synthetic DNA

    (iii) FEATURES:      N is A, G, C, or T

    (xi) SEQUENCE DESCRIPTION:  SEQ ID NO: 5:

CGTGGSCMTS STGGGCAACN YCCTG          25

(2) INFORMATION FOR SEQ ID NO: 6:

    (i)   SEQUENCE CHARACTERISTICS:
        (A) LENGTH:       27
        (B) TYPE:        Nucleic acid
        (C) STRANDEDNESS: Single
        (D) TOPOLOGY:    Linear

    (ii) MOLECULE TYPE:    Other nucleic acid
                       Synthetic DNA

    (iii) FEATURES:      N is A, G, C, or T

```
(xi) SEQUENCE DESCRIPTION:  SEQ ID NO: 6:

GTNGWRRGGC ANCCAGCAGA KGGCAAA          27

(2) INFORMATION FOR SEQ ID NO: 7:

        (i)   SEQUENCE CHARACTERISTICS:
              (A) LENGTH:        24
              (B) TYPE:          Nucleic acid
              (C) STRANDEDNESS:  Single
              (D) TOPOLOGY:      Linear
        (ii) MOLECULE TYPE:      Other nucleic acid
                                 Synthetic DNA

(xi) SEQUENCE DESCRIPTION:  SEQ ID NO: 7:

TCGGCTTCTC CATCAAGAGG ACCC              24

(2) INFORMATION FOR SEQ ID NO: 8:

        (i)   SEQUENCE CHARACTERISTICS:
              (A) LENGTH:        24
              (B) TYPE:          Nucleic acid
              (C) STRANDEDNESS:  Single
              (D) TOPOLOGY:      Linear

        (ii) MOLECULE TYPE:      Other nucleic acid
                                 Synthetic DNA

(xi) SEQUENCE DESCRIPTION:  SEQ ID NO: 8:

CACGCTGCGC ACATAGTGGG CGAA              24
```